# EUROPÄISCHE PATENTANMELDUNG

(11) **EP 1 867 638 A1**
(43) Veröffentlichungstag der Anmeldung: **19.12.2007**
(21) Anmeldenummer: 06012414.6
(22) Anmeldetag: 16.06.2006
(51) Int. Cl.: C07D 237/26, C07D 221/22, C07D 471/04, C07D 491/04, C07D 491/08, C07H 17/00, C07K 7/00

(54) **Verfahren zur kovalenten Verknüpfung zweier Moleküle mittels Diels-Alder-Reaktion mit inversem Elektronenbedarf**

(71) Anmelder: Deutsches Krebsforschungszentrum Stiftung des öffentlichen Rechts, 69120 Heidelberg (DE)
(72) Erfinder: Wießler, Manfred, 67227 Frankenthal (DE); Müller, Eduard, 68723 Schwetzingen (DE); Lorenz, Peter, 69221 Dossenheim (DE); Kliem, Cristian, 64646 Heppenheim (DE); Fleischhacker, Heinz, 69221 Dossenheim (DE)
(74) Vertreter: Schüssler, Andrea

(57) **Zusammenfassung**

Die vorliegende Erfindung betrifft ein Verfahren zur Verknüpfung zweier Moleküle mittels Diels-Alder-Reaktion mit inversem Elektronenbedarf, das die folgenden Schritte aufweist:
Umsetzung eines
(a) Triazins oder Tetrazins mit einem oder mehreren elektronenziehenden Substituenten am Ring als Dienkomponente, wobei die elektronenziehenden Substutuenten ausgewählt sind aus:
-COOR
- C(O)NR₂
-CX₃(X=Halogen)
- Halogen
-CN
-SO₂-R oder SO₃-
-PR₂
mit R = H, Alkyl, Aryl-, Heterocyclus, wobei diese wiederum ggf. substituiert sein können mit Alkyl-, OH-, SH-, Halogen-, Aryl-, Heterocyclus, Nitro-, Carboxyamido-, oder Amin-Gruppe..
mit
(b) einer isolierten Doppelbindung bzw. Dreifachbindung in einem (hetero)carbocyclischen Ring oder einer isolierten olefinischen Doppelbindung bzw. Dreifachbindung in einer linearen oder verzweigten Kohlenwasserstoffkette, die ggf. Heteroatome enthalten kann, als Dienophilkomponente

## Beschreibung

Die vorliegende Erfindung betrifft ein Verfahren zur kovalenten Verknüpfung zweier Moleküle durch Diels-Alder-Reaktion mit inversem Elektronenbedarf.

Bei Untersuchungen von biologischen Systemen stoßen Molekularbiologen und Chemiker immer wieder auf die Notwendigkeit, zwei molekulare Einheiten kovalent miteinander verknüpfen zu müssen, etwa ein Oligosaccharid mit einem Peptid, ein Reportermolekül mit einem Biopolymer, zwei Biopolymere miteinander oder ein niedermolekulares Therapeutikum mit einem Biopolymer. Meistens verfügen alle die genannten Verbindungen über eine Vielzahl chemischer Funktionen, deren jeweiliges Reaktionsverhalten unter den Bedingungen der Ligationsreaktion beachtet werden muß. Daraus folgt, dass chemoselektive Ligationsreaktionen über einem eindeutigen Reaktionsverlauf verfügen sollten, ohne dass die anderen vorhandenen chemischen Funktionen oder Gruppen angegriffen werden oder in das Reaktionsgeschehen aktiv eingreifen. Dieses Vorhaben ist nur dann zu verwirklichen, wenn im Ligationsschritt zwei selektiv miteinander reagierende funktionelle Gruppen beteiligt sind. Weiterhin wäre wünschenswert, dass eine solche Ligations-Reaktion ohne Verwendung von Schutzgruppen in jedem Milieu und bei einem dem jeweiligen Biopolymer angepassten pH Wert ablaufen kann.

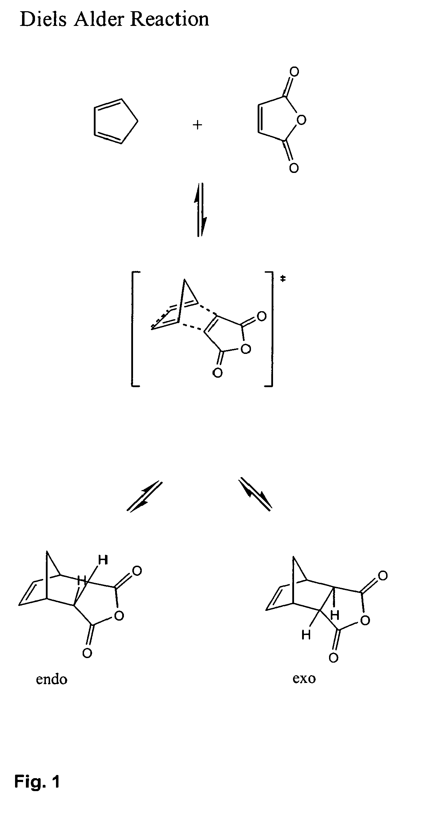

Eine der wenigen chemischen Reaktionen, die alle diese Bedingungen voll erfüllen kann, ist die Cycloaddition, entweder der 4+2 Typ, bekannt als Diels-Alder-Reaktion (Fig. 1; J. Sauer, 1966, Angew. Chem.78, 233), oder der 3+2 Typ, bekannt als 1,3 dipolare Cycloaddition. Eine weitere in den letzten Jahren weiter entwickelte Methode ist die Staudinger-Ligation (Review: Angew.Chem 2004, 116, 3168-3178).

In der DE-A-100 41 221.1 wurde die Anwendung der klassischen Diels Alder Reaktion als Ligationsreaktion, wobei das Dien mit elektronenspendenden und das Dienophil mit elektronenziehenden Substituenten versehen ist, gezeigt. Als Diene wurden dabei Furan und seine Derivate und als Dienophile substituierte Maleinimide eingesetzt. Dieses System wurde wegen der einfachen Zugänglichkeit der jeweiligen Komponenten und deren einfacher Chemie ausgewählt. Viele Furane sind leicht aus Sacchariden herstellbar und stehen in größeren Mengen zur Verfügung. Wie viele chemische Reaktionen ist auch die Diels Alder Reaktion (im nachfolgenden "DAR" genannt) umkehrbar, vor allem bei höheren Temperaturen. Diese Reversibilität ist besonders in dem System Furan/Maleinimid ausgeprägt, was durch die hohe Reaktivität der Maleinimide für nukleophile Additionen hervorgerufen wird. Dies lässt sich an der Verwendung von Maleinimiden zur Markierung von Peptiden oder zu ihrer Verknüpfung leicht ablesen. Dabei addiert sich die Thiolgruppe des Proteins in einer sehr schnellen, irreversiblen Reaktion an die Doppelbindung des Maleinimids. Bereits die geringen Mengen an Maleinimid, die durch die Rückreaktion der DAR im Gleichgewicht vorhanden sind, werden durch eine solche Addition abgefangen und verschieben damit das Gleichgewicht auf die Seite der Ausgangsstoffe. Dies ist ein echter Nachteil der DAR, da dadurch die Ausbeute des gewünschten Produkts signifikant minimiert wird.

Die Aufgabe der vorliegenden Erfindung besteht daher darin, ein Verfahren zur Verfügung zu stellen, mit dem auch kompliziert aufgebaute Verbindungen kovalent miteinander verknüpft werden können und Bibliotheken aufgebaut werden können.

Diese Aufgabe wird durch die Gegenstände der Patentansprüche gelöst.

Die Aufgabe wird durch eine Diels-Alder-Reaktion mit inversem Elektronenbedarf gelöst, die die folgenden Schritte aufweist:
Umsetzung eines
   (a) 1,2,4-Triazins oder 1,2,4,5-Tetrazins oder eines 1,2-Diazins mit einem oder mehreren elektronenziehenden Substituenten am Ring als Dienkomponente, wobei die elektronenziehenden Substutuenten ausgewählt sind aus:
      - COOR
      - C(O)NR₂
      - CX₃ (X = Halogen)
      - Halogen
      - CN
      - SO₂-R oder SO₃-R
      - PR₂
      mit R = H, Alkyl, Aryl-, Heterocyclus, wobei diese wiederum ggf. substituiert sein können mit Alkyl, OH, SH, Halogen, Aryl-, Heterocyclus, Nitro-, Carboxyamido-, oder Amin-Gruppe. mit
   (b) einer isolierten Doppelbindung bzw. Dreifachbindung in einem (hetero)carbocyclischen Ring oder einer isolierten olefinischen Doppelbindung bzw. Dreifachbindung in einer linearen oder verzweigten Kohlenwasserstoffkette, die ggf. auch Heteroatome enthalten kann, als Dienophilkomponente

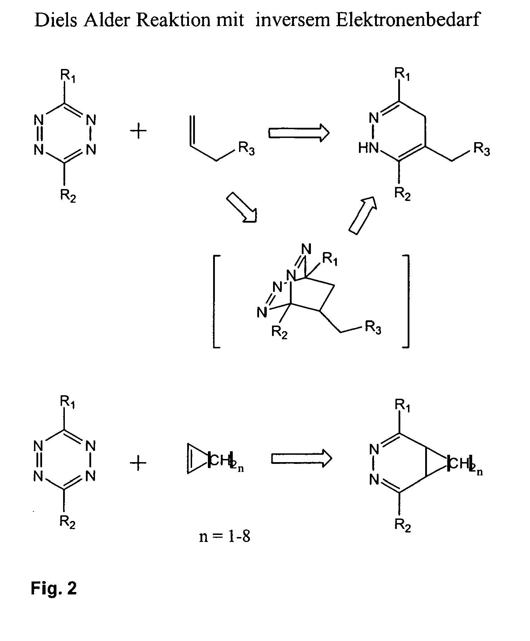

Die Erfinder haben sich solchen Diels-Alder-Reaktionen zugewandt, die unter Abspaltung eines Molekülteils verlaufen und damit das Gleichgewicht der Reaktion vollständig auf die Seite des Produktes verschieben. Sofern der abgespaltene Molekülteil flüchtig ist, wird eine Rückreaktion unmöglich gemacht. Neben einigen speziellen Dienen im Rahmen der klassischen DAR, wie z.B. dem Cyclopentadienon, wird dieser Reaktionstyp vor allem von der Diels Alder Reaktion mit inversem Elektronenbedarf repräsentiert (Fig. 2). Diese Reaktion ist gut untersucht und hat vor allem Eingang in die Heterocyclen-Synthese gefunden. Bei dieser Art von DAR werden die Art der Substituenten im Dien und im Dienophil, wie sie von O. Diels und K. Alder definiert wurden, umgekehrt oder invertiert. Jetzt wird das Dien mit Elektronen-ziehenden Substituenten versehen und damit elektronenarm, während das Dienophil durch seine Substitution nunmehr elektronenreich wird. Bereits die DAR des Tetrachlorocyclopentadiens mit Olefinen wird als DAR mit inversem Elektronenbedarf charakterisiert (im nachfolgenden mit "DARinv" bezeichnet). So können solche Diels-Alder-Reaktionen mit inversem Elektronenbedarf bereits bei Raumtemperatur vollständig ablaufen.

Die bereits zu Beginn definierten Kriterien für die Entwicklung einer effektiven Ligationsreaktion werden somit von der der inversen DAR in nahezu idealer Weise erfüllt. Voraussetzung dafür ist allerdings die Synthese geeignet funktionalisierter 1,2,4,5-Tetrazine, 1,2,4-Triazine und 1,2-Diazine als Diene und von Olefinen als Dienophilen. Da solche Verbindungen bisher nicht bekannt sind, liegt die Ausarbeitung dieser Synthesen für den jeweiligen Verwendungszweck dieser Anmeldung zugrunde. Beide Varianten der DARinv als Ligationsreaktion werden verifiziert: zum einen die Einführung des Diens in das Zielmolekül, z.B. ein Peptid, gefolgt von der Umsetzung mit dem als Dienophil ausgeprägtem Saccharid, als auch die umgekehrte Vorgehensweise. Sowohl das Dien als auch das Dienophil sollen maximale Reaktivität bei maximaler Stabilität besitzen, um die DARinv möglichst in allen Lösungsmitteln bei Raumtemperatur durchführen zu können.

### Dien-Komponente

Ziel bei der Synthese geeigneter Diene zur Funktionalisierung von Peptiden, Oligonukleotiden, von Oberflächen oder von Therapeutika ist die Darstellung von symmetrisch oder unsymmetrisch substituierten 1,2,4,5-Tetrazinen, 1,2,4-Triazinen und 1,2-Diazinen, die einfach in die genannten Bio-Moleküle eingebaut werden können. Der bereits bekannte 3,6-Dicarbonsäureester des 1,2,4,5-Tetrazins ist als Ausgangsprodukt zur Darstellung von geeignet funktionalisierten 1,2,4,5-Tetrazinen als Diene nicht gut geeignet. Zum einen besitzt diese Verbindung, die prächtig rote Kristalle bildet, in alkoholischen und wässrigen Lösungsmitteln nicht die ausreichende Stabilität, zum anderen sind nukleophile Substitutionsreaktionen an den Estergruppen nicht möglich, da unter diesen Bedingungen das Molekül zerfällt. (Kämpchen T. et al 1982, Chem. Ber. ,115, 683-694.)

Bei der dreistufigen Synthese dieses Tetrazins, beginnend mit Diazoessigester wird jedoch die Stufe des Dihydro-1,2,4,5-Tetrazin-dicarbonsäureesters durchlaufen. An dieser Substanz lassen sich nukleophile Substitutionsreaktionen, bedingt durch die große Reaktivität der Estergruppen, problemlos durchführen. Da die zweite nukleophile Substitution, vor allem mit sekundären Aminen, langsamer abläuft als die erste, gelingt es auch Monoamide herzustellen. Dies ist auch ein, wichtiger Schritt zur Herstellung monofunktionalisierter Tetrazine zur Derivatisierung von Peptiden. Weitere Möglichkeiten zur Darstellung von monofunktionalisierten Tetrazinen werden weiter unten aufgezeigt. Gleichzeitig eröffnet dies auch die Darstellung unsymmetrischer Tetrazindiamide.

Ein Syntheseweg zur Darstellung von Triazinen besteht in der Umsetzung von 1,2-Diketo-Derivaten mit Amiddrazonen. Auf diesem Weg kann auch der Tricarbonsäureester des 1,2,4-Triazins in größeren Mengen hergestellt werden. Bisher ist es nicht gelungen bei der Umsetzung mit Aminen zwischen den Esterfunktionen zu diskriminieren. Für eine gezielte Einführung des Triazin-Restes in beliebige Moleküle ist eine Esterfunktion ausreichend, zur Erhaltung der Dien-Aktivität des Triazins sollten dann jedoch ausreichend elektronegative Substituenten zugegen sein.

Triazine-1,2,4 sind im allgemeinen weniger reaktiv in der DARinv als die Tetrazine, doch reicht ihre Reaktionsgeschwindigkeit noch für Ligationsreaktionen aus, vor allem wenn sie mit einem sehr reaktiven Dienophil umgesetzt werden.

Noch geringere DARinv Aktivität als Diene besitzen die 1,2-Diazine, die durch Oxidation der bei der DARinv von Tetrazinen mit Olefinen entstehenden Dihydropyridazine gebildet werden. Sie entstehen direkt bei der DARinv von Tetrazinen mit Dreifachbindungen oder mit Enaminen. Dadurch bietet sich auch die Möglichkeit in einer Sequenz, ausgehend von einem Tetrazin über das Diazin durch zwei aufeinander folgende DARinv Reaktionen, unterbrochen durch die Oxidation des Dihydropyridazins zum Pyridazin, zwei beliebige Moleküle, die als Dienophile ausgebildet sind in vorgegebener Weise miteinander zu verknüpfen.

Erfindungsgemäß eignen sich als Dien-Komponente Tetrazine, Triazine, und Diazine welche mit elektronenziehenden funktionellen Gruppen ein- oder mehrfach substituiert sein können. Diese elektronenziehenden funktionellen Gruppen können ausgewählt sein aus:
- COOR (bevorzugt: COOH)
- C(O)NR₂
- CX₃ (X = Halogen) (bevorzugt CF₃)
- Halogen (F, Cl, Br oder I)
- CN
- SO₂-R oder SO₃-R
- PR₂

Die funktionellen Gruppen R, welche bevorzugt eine Funktionalität zur Anknüpfung an weitere Moleküle (z.B. an Peptide, Sacharide oder Nukleinsäuren) bereitstellen, können ausgewählt sein aus H, Alkyl, Aryl-, Heterocyclus, wobei R wiederum ggf. substituiert sein kann mit Alkyl-, OH-, SH-, Halogen-, Aryl-, Heterocyclus, Nitro-, Carboxyamido-, oder Amin-Gruppe..

"Alkyl" bedeutet C₁- C₂₀, bevorzugt Methyl, Ethyl, iso-Propyl, Tert.-Butyl usw., Die Aryl- bzw. Heterocyclus-Substituenten können ausgewählt sein aus: Phenyl-, - Thienyl-, Thiophenyl-, Furyl-, Furanyl-, Cyclopentadienyl-,Pyranyl-, Pyrrolyl-, Imidazolyl-, Pyrazolyl-, Pyridyl-, Pyrazinyl-, Pyrimidinyl-, Pyrazinyl-, Pyridazinyl-, Thiazolyl-, Oxazolyl-, Indolyl-, Furazannyl-, Pyrrolinyl-, Imidazolinyl-, Pyrazolinyl-, Thiazolinyl-, Triazolyl-, Tetrazolyl-Gruppe, sowie die Positionsisomeren des oder der Heteroatome, die diese Gruppen umfassen können, ein Rest bestehend aus carbocyclischen kondensierten Ringen, beispielsweise die Naphthylgruppe oder die Phenanthrenylgruppe, ein Rest bestehend aus kondensierten heterocyclischen Ringen, beispielsweise Benzofuranyl, Benzothienyl, Benzimidazolyl, Benzothiazolyl, Naphtho[2,3-b]thienyl, Thianthrenyl, lsobenzofuranyl, Chromenyl, Xanthenyl, Phenoxathionyl, Indolizinyl, Isoindolyl, 3H-Indolyl, Indolyl, Indazolyl, Purinyl, Chinolizinyl, Isochinolyl, Chinolyl, Phthalzinyl, Naphthyridinyl, Chinoxalinyl, Chinazolinyl, Chinolinyl, Pteridinyl, Carbazolyl, β-Carbolinyl, Cinnolinyl, Acridinyl, Phenazinyl, Phenothiazinyl, Phenoxazinyl, Indolinyl, Isoindolinyl, Imidazopyridyl, Imidazopyridmidinyl oder auch die kondensierten polycyclischen Systeme bestehend aus heterocyclischen Monozyklen, wie beispielsweise vorstehend definiert, wie beispielsweise Thionaphthenyl, Furo[2,3-b]pyrrol oder Thieno[2,3-b]furan, und insbesondere die Phenyl-, Furylgruppen, wie 2-Furyl, Imidazolyl, wie 2-Imidazolyl, Pyridyl, wie 2-Pyridyl, 3-Pyridyl, 4-Pyridyl, Pyrimidinyl, wie Pyridmid-2-yl, Thiazolyl, wie Thiazol-2-yl, Thiazolinyl, wie Thiazolin-2-yl, Triazolyl, wie Triazolyl-2-yl, Tetrazolyl, wie Tetrazol-2-yl, Benzimidazolyl, wie Benzimidazol-2-yl, Benzothiazolyl, Benzothiazol-2-yl, Purinyl, wie Purin-7-yl, oder Chinolyl, wie 4-Chinolyl.

Die Dien-Komponente kann aber an einer oder mehreren Positionen auch Aminosäure-, Peptid-, Saccharid-, Lipid- oder Oligonukleotid- bzw. Nukleinsäure-Substituenten tragen. An die Dien-Komponente lassen sich auch alle Arten pharmazeutischer Wirkstoffe, Markierungen, Farbstoffe, Komplexe (z.B. Carboran, Ferrocen), Quantumdots, Chelat-/Kompexbildner, Diagnostika oder Therapeutika koppeln und Kombinationen davon

Bevorzugte Dien-Komponenten sind alle Ester der dargestellten Tetrazine, Triazine und Diazine und die davon abgeleiteten Verbindungen: z. B. Tetrazinmonoamide, Tetrazin-diamide, Tetrazin-3-trifluormethyl-6-carbonsäureamide, Triazin-tricarbonsäure-mono-, di-, und tri-amide , 3-Carboxyamid-5,6-bis-trifluormethyl-triazin 1,2,4) 1,2-Diazine 3,6,dicarbonsäureamide. Ebenso können natürlich auch deren Homologe mit Ethyl- oder Propylgruppe statt Methyleingesetzt werden. Die erwähnten Tetrazine sind relativ leicht durch Oxidation aus den entsprechenden Dihydroverbindungen herzustellen, welche über den Dihydrotetrazin-dicarbonsäureester zugänglich sind, welcher wiederum in zwei Stufen aus dem käuflichen Diazoessigester leicht hergestellt werden kann

### Dienophil-Komponente

Als Dienophil für die DARinv genügt eine endständige olefinische Doppelbindung ohne zusätzliche Aktivierung, eine Funktion, die in biologischen Systemen nicht oder sehr selten vorkommt. Eine zweifach substituierte Doppelbindung, wie sie in Fettsäuren oder Lipiden vorkommt, reagiert unter Normalbedingungen nur sehr langsam im Sinne der DARinv. Auch eine Methylen-Gruppe wie im Perillaalkohol reagiert nur sehr langsam bei Raumtemperatur (60 Stunden) im Sinne der DARinv unter Bildung des erwarteten Adduktes. Sobald eine Doppelbindung allerdings in einem carbocyclischen Ringsystem integriert ist, reagiert sie wesentlich schneller als Dienophil, wobei die Reaktivität mit zunehmender Ringgröße, beginnend beim Cyclopropen, abnimmt und beim Cyclohexen ein Minimum durchläuft. In Ringsystemem größer als Siebenringe können auch Dreifachbindungen als inverse Dienophile reagieren.

Unter einem carbocyclischen Ring wird erfindungsgemäß jeder mono-, bi- oder tricyclische Kohlenstoffring verstanden. In diesen Ringen können auch Heteratome enthalten sein. (N, O, S, Si)

Als Dienophil-Komponente kommt auch eine isolierte olefinische Doppelbindung bzw. Dreifachbindung in einer linearen oder verzweigten Kohlenwasserstoffkette, die ggf. auch Heteroatome (N; O, S, Si) enthalten kann, in Frage.

Da sich die Reaktionsgeschwindigkeiten verschiedener Dienophile gegenüber dem gleichen Dien in der DARinv um Zehnerpotenzen unterscheiden, können bei Gegenwart von zwei unterschiedlichen Dienophilen in einem Templat zielgerichtete Reaktionen durchgeführt werden. So beschreibt Sauer (Eur.J.Org.Chem 1998, 2885-2896) einen Unterschied in der Reaktivität zwischen dem Cyclobuten und Cyclopenten um den Faktor 12 gegenüber dem unsubstituerten Tetrazin, während zwischen Cyclopenten und Cyclohexen dagegen ein Faktor 1200 beobachtet wird. Daraus folgt, dass bei Gegenwart eines Cyclobutens und eines Cyclohexens im gleichen Molekül ein Unterschied in der Reaktionsgeschwindigkeit etwa um den Faktor 1200 zu beobachten ist. Bezogen auf die Ausbeute bedeutet dies etwa eine Verunreinigung von etwa einem Promille. Verschieden substituierte Tetrazine, Triazine und Diazine verfügen als Diene natürlich auch über unterschiedliche Reaktivitäten gegenüber dem gleichen Dienophil, so dass sich Reaktivitätsskalen von Dienen und Dienophilen definieren lassen, die sehr spezifische und gezielte mehrfache Reaktionen zulassen. Erweitert man dieses Schema noch um die klassische Diels Alder Reaktion, die sich hinsichtlich der elektronischen Anforderungen an Dien und Dienophil in Bezug auf die inverse Variante absolut orthogonal verhält, so entsteht ein sehr variables und leistungsfähiges Netzwerk von Ligationsreaktionen. Oftmals wird durch eine vorgeschaltete klassische DAR erst das Dienophil für eine DARinv geschaffen. Ein sehr schönes Beispiel für diese Sequenz ist die DAR zwischen Cyclopentadien und MSA (Maleinsäureanhydrid) unter Bildung des Norbornenanhydrides oder die DAR zwischen Cyclooctatetraen und MSA, bei der ein Cyclobuten und ein Cyclohexen im gleichen Molekül gebildet werden. Da sich sowohl das Norbornenanhydrid als auch das bicyclische COT-Anhydrid mit Aminen leicht weiter derivatisieren lassen, können sie leicht in funktionelle, für die Ligation geeignete Moleküle überführt werden. Durch die Metathese-Reaktion lassen sich ebenfalls Ringsysteme mit Doppelbindungen generieren. Auch durch photochemische Ringschluss-Reaktionen von cyclischen 1,3-Dienen lassen sich Cyclobutene als reaktive Dienophile erzeugen, so dass die auf der DARinv basierende Ligationstechnik mit der Photolithographie verknüpft werden kann.

In der Literatur ist beschrieben, dass sich die Oberflächen von Kohlenstoff (Diamant, Fullerenen, Carbonanoröhren), Germanium und Silizium wie Dienophile bei der klassischen DAR verhalten. (Roucoules V. et al, 2005, Langmuir 21, 1412-1415). Sie reagieren mit Cyclopentadien unter Bildung des Norbornen-Ringsystems, wobei wiederum ein für die DARinv geeignetes Dienophil entsteht. Damit lassen sich diese Oberflächen problemlos durch die DARinv funktionalisieren. In der Verbindung mit der photochemischen Aktivierung durch Cyclisierung von 1,3-Dienen zu Cyclobutenen ergeben sich durch die Abfolge : 1.) Kovalente Verankerung eines cyclischen 1,3-Diens an einer Oberfläche oder einem Halbleiter 2.) Photochemische Cyclisierung zum Cyclobuten, 3.) DARinv mit einem Dien, an das z.B. ein Protein oder ein Saccharid kovalent geknüpft ist, vollkommen neuartige räumliche Funktionalisierungmöglichkeiten.

Bevorzugte Dienophil-Komponenten sind Säuren und Anhydride, die davon abgeleiteten funktionalisierten Imide und Amide und deren Reduktionsprodukte, sowie die zugehörigen Ester und deren Substitutions- und Reduktionsprodukte, die eine gespannte oder eine endständige Doppelbindung enthalten, z.B. exo- oder endo-Norbornendicarbonsäureanhydrid, Cyclobutendicarbonsäureanhydrid, Cyclohexendicarbonsäureanhydrid., das einfach zugängliche bicyclische COT-MSA Addukt, das zudem den Vorteil aufweist, zwei unterschiedlich reaktive Doppelbindungen zu enthalten, nämlich einen Cyclobuten- und einen Cyclohexen-Ring. Das gleiche gilt für den 2-Allyl-2-Propargyl-malonester. Weitere bevorzugte Dienophil-Komponenten sind Dehydroprolin, Allyl-Prolin, Allylmalonester, Allylgalactose, Allyl-Silsesquioxan, sowie alle Verbindungen die eine Allyl, Butenyl oder Pentenyl-Gruppe tragen. Die Dienophil-Komponente kann ggf. auch mit funktionellen Gruppen ein- oder mehrfach substituiert sein. Die funktionellen Gruppen können ausgewählt sein aus beispielsweise Alkylketten (C₂ - C₂₀, bevorzugt Methyl, Ethyl, iso-Propyl, Tert.-Butyl usw., ggf. Halogen-substituiert), OH, SH, Halogene, Aryl-, Carboxyl-, Carbonyl-, Nitro-, Carboxyamido-, Keto-, Sulfoxid-, Sulfon-, Sulfonsäure-, Sulfid-, Sulfat-, Phosphorsäure- oder Amino-Gruppen, die direkt oder über Alkylreste gebunden sind. Die Dienophil-Komponente kann auch aromatische oder heterozyklische Reste enthalten. Diese können ausgewählt sein aus: Phenyl-, Thienyl-, Thiophenyl-, Furyl-, Cyclopentadienyl-, Furanyl-, Pyranyl-, Pyrrolyl-, Imidazolyl-, Pyrazolyl-, Pyridyl-, Pyrazinyl-, Pyrimidinyl-, Pyrazinyl-, Pyridazinyl-, Thiazolyl-, Oxazolyl-, Indolyl-, Furazannyl-, Pyrrolinyl-, Imidazolinyl-, Pyrazolinyl-, Thiazolinyl-, Triazolyl-, Tetrazolyl-Gruppe, sowie die Positionsisomeren des oder der Heteroatome, die diese Gruppen umfassen können, ein Rest bestehend aus carbocyclischen kondensierten Ringen, beispielsweise die Naphthylgruppe oder die Phenanthrenylgruppe, ein Rest bestehend aus kondensierten heterocyclischen Ringen, beispielsweise Benzofuranyl, Benzothienyl, Benzimidazolyl, Benzothiazolyl, Naphtho[2,3-b]thienyl, Thianthrenyl, Isobenzofuranyl, Chromenyl, Xanthenyl, Phenoxathionyl, Indolizinyl, Isoindolyl, 3H-Indolyl, Indolyl, Indazolyl, Purinyl, Chinolizinyl, Isochinolyl, Chinolyl, Phthalzinyl, Naphthyridinyl, Chinoxalinyl, Chinazolinyl, Chinolinyl, Pteridinyl, Carbazolyl, β-Carbolinyl, Cinnolinyl, Acridinyl, Phenazinyl, Phenothiazinyl, Phenoxazinyl, Indolinyl, lsoindolinyl, Imidazopyridyl, Imidazopyridmidinyl oder auch die kondensierten polycyclischen Systeme bestehend aus heterocyclischen Monozyklen, wie beispielsweise vorstehend definiert, wie beispielsweise Thionaphthenyl, Furo[2,3-b]pyrrol oder Thieno[2,3-b]furan, und insbesondere die Phenyl-, Furylgruppen, wie 2-Furyl, Imidazolyl, wie 2-Imidazolyl, Pyridyl, wie 2-Pyridyl, 3-Pyridyl, 4-Pyridyl, Pyrimidinyl, wie Pyridmid-2-yl, Thiazolyl, wie Thiazol-2-yl, Thiazolinyl, wie Thiazolin-2-yl, Triazolyl, wie Triazolyl-2-yl, Tetrazolyl, wie Tetrazol-2-yl, Benzimidazolyl, wie Benzimidazol-2-yl, Benzothiazolyl, Benzothiazol-2-yl, Purinyl, wie Purin-7-yl, oder Chinolyl, wie 4-Chinolyl.

Die vielfältigen möglichen Substitutionen im Dien und Dienophil lassen es zu, dass ein Dien, das in der DARinv aktiv ist, entweder mit einer Dien-Struktur oder mit einem Dienophil der klassischen DAR im gleichen Molekül enthalten sein kann. Das gleiche gilt naturgemäß auch für das inverse Dienophil. Dadurch ergeben sich eine Vielzahl von gezielten Ligationsmöglichkeiten durch gleichzeitig ablaufende DAR und DARinv.

Die Dienophil-Komponente kann aber an einer Seite auch Aminosäure-, Peptid-, Saccharid-, Lipid- oder Oligonukleotid- bzw. Nukleinsäure-Substituenten tragen. An die Dienophil-Komponente lassen sich auch alle Arten pharmazeutischer Wirkstoffe, Markierungen, Farbstoffe, Komplexe (z.B. Carboran, Ferrocen), Quantumdots, Chelat-/Kompexbildner, Diagnostika oder Therapeutika koppeln.

Orientierende kinetische Messungen der DARinv für die hergestellten Diene und Dienophile zeigen das erwartete breite Reaktionsverhalten.

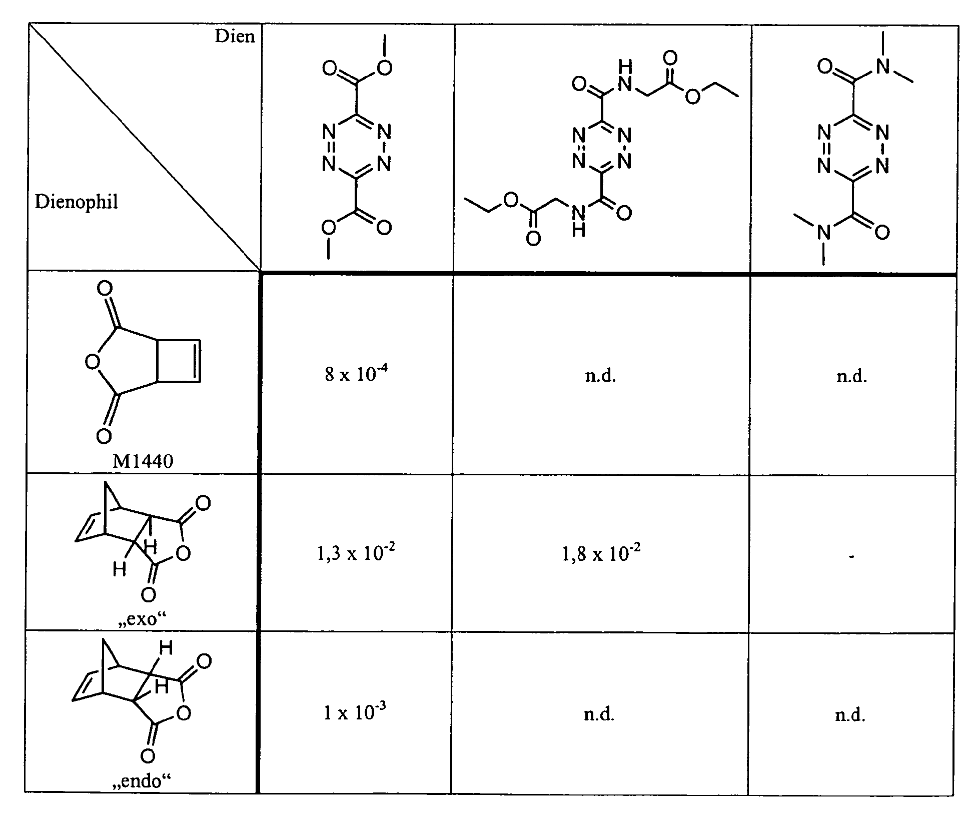

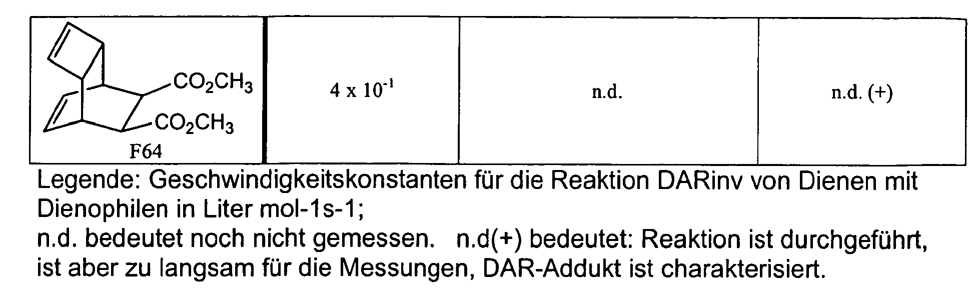

Diese kinetischen Daten zeigen sehr deutlich, dass das tricyclische Anhydrid aus COT und MSA und seine Derivate außergewöhnlich reaktive Dienophile für die DARinv darstellen. Zum Vergleich: die Reaktion zwischen Furan und Maleinimid zum endo-Addukt weist eine Geschwindigkeitskonstante von 0,007 auf und für das exo-Addukt eine solche von 0,00002 Liter/mol x s. (Vogel P. et al 1999, Tetrahedron, 55, 13521, Report 510). Einfache Modellrechnungen zeigen, dass der Cyclobutenring in diesem Tricyclus sehr gut zugänglich ist, eine Voraussetzung für die enorm hohe Reaktionsgeschwindigkeit. Obwohl demgegenüber das Cyclobuten-dicarbonsäurenahydrid eine um den Faktor 500 geringere Reaktionsgeschwindigkeit aufweist, lässt sich nach 24 Stunden das entsprechnende Addukt quantitativ isolieren. Das ist auf die Interaktion der Carbonylgruppen des Anhydridringes mit den Carbonylgruppen des Diens im Übergangszustand zurückzuführen. Belegt wird dies durch die hohe Dienophil-aktivität in der DARinv des Bicyclo 2.2.0-hexen-5-dicarbonsäureanhydrides-2,3 bei dem der Anhydridring im Übergangszustand weiter entfernt ist. Mit diesen Überlegungen stimmt auch der beobachtete Unterschied in der Reaktiongeschwindigkeit zwischen dem exo- und dem endo-Norbornenanhydrid überein. Auch die Diamide des Tetrazins besitzen die gleiche Dien Aktivität in der DARinv wie der Diester selbst. Diese gezeigten Unterschiede lassen sich sehr gut zur Durchführung selektiver Reaktionen ausnützen, wenn in einem Molekül zwei Diene oder zwei Dienophile mit deutlichen Unterschieden in der Reaktionsgeschwindigkeit enthalten sind..

Die inverse Diels-Alder-Reaktion ist eine Reaktion bei der relativ niedrige Aktivierungsenergien zu überwinden sind, so daß diese Reaktionen bereits bei Raumtemperatur oder leicht erhöhten Temperaturen ablaufen können. Sie besitzt eine starke Temperaturabhängigkeit, was auf eine stark negative Entropie hindeutet, wie es für eine DAR erwartet werden kann. Diels-Alder-Reaktionen können durch hohen Druck beschleunigt werden. Gerade in den letzten Jahren sind eine Reihe von Katalysatoren bekannt geworden, die in der Lage sind, unter milden Bedingungen effektiv Diels-Alder Reaktionen zu katalysieren (K. Pindur et al., Chem Rev. 1993, 93, S. 741-761; Kündig et al., Angew. Chem. 1999, 111, S. 1298-1301). Auch Ultraschall wirkt beschleunigend auf die DAR. Hinsichtlich der Ausbeuten bietet die inverse Diels-Alder Reaktion große Vorteile, da sie ohne weitere Nebenprodukte und mit nahezu quantitativer Ausbeute verläuft. Die inverse Diels-Alder Reaktion wird daher von den Erfindern eingesetzt, um kompliziert aufgebaute biologische Moleküle und Bibliotheken aufzubauen. Bei geschickter Substitution der beiden Ausgangsverbindungen (Dien und Dienophil) mit funktionellen Gruppen oder Resten sind damit Moleküle zugänglich, die drei oder sogar vier unterschiedliche Reste enthalten können.

Mittels der DARinv können verschiedenste Moleküle miteinander verknüpft oder aneinander ligiert werden, auch mehrfach oder in sequentieller Folge.. Beispielsweise sind dies Aminosäuren, Peptide, Proteine, Antikörper, Saccharide, Nukleinsäuren, Nukleoside, Festphasen-Oberflächen, Nanoteilchen, Farbstoffe, Therapeutika, Diagnostika, Chelat-/Komplexbildner, Quantumdots., Membranen, Oberflächen, Halbleiter.

Der Begriff "Peptid" bzw. "Protein" umfasst Peptide bzw. Proteine jeglicher Länge und Komplexität, auch Glycokonjugate.

Der Begriff "Saccharid" umfaßt Saccharide jeglicher Art, insbesondere Mono-, Di-, Oligo- oder Polysaccharide (z.B. mono-, di- tri-, multi-antennäre sowie dendritische Saccharide) in allen stereoisomeren und enantiomeren Formen. Diese können Pentosen oder Hexosen sein, welche in der L- oder D-Form vorliegen. Als Monosaccharide sind insbesondere Glucose, ganz besonders α- und β- D-Glucose, Fructose, Galactose, Mannose, Arabinose, Xylose, Fucose, Rhamnose, Digitoxose und Derivate davon bevorzugt. Als Disaccharide eignen sich insbesondere Saccharose, Maltose, Laktose oder Gentobiose, entweder 1,4- oder 1,6-verknüpft, sowie Derivate davon. Als Saccharide gelten hier auch Zuckeralkohole, Polyole, Inosite und Derivate davon, ganz besonders cis-Inositol, epi-Inositol, allo-Inositol, myo-Inositol, muco-Inositol, chiro-Inositol, neo-Inositol, scyllo-Inositol, Pinpollitol, Streptamin, Quercitol, Chinasäure, Shikimisäure, Conduritol A bzw. B, Validatol und Quebrachitol, z.B. aus Galactinolen, sowohl aus pflanzlichen Quellen, wie Zuckerrüben (daraus erhältlich: Hydroxymethylfurfural; F.W. Lichtenthaler, Mod. Synth. Meth. 1993, 6, S. 273-376), als auch aus Milchprodukten, oder durch enzymatische Enantiomerentrennung gewonnene Verbindungen. Ferner sind erfindungsgemäß einsetzbare Saccharide Glycokonjugate. Diese können Konjugate von z.B. Sacchariden mit Peptiden, Lipiden, Säuren (--> Ester), Alkylresten (---> Ether), Heterozyklen oder anderen Kohlenhydraten sein. Ein Beispiel von Glycokonjugaten ist Z1-Z10, ein Gemisch von 10 Glykokonjugaten. Bei den Verbindungen Z1-Z10 handelt es sich um in der Natur vorkommende Glycopeptide, Glycoproteine und Lipopolysaccharide. Derivate der erwähnten Saccharide sind z.B. mit Schutzgruppen (z.B. Benzoyl-, Silyl-, Dimethoxytrityl-) geschützte Saccharide und/oder mit funktionellen Gruppen, wie Amino-, Nitro-, Sulfat-, Carboxy-, Carboxyamido-, Keto-, Sulfoxid-, Sulfon-, Sulfonsäure-, Phosphorsäure-, Phosphonsäure-, Mono/Di/Trialkylamidgruppen oder Halogenidgruppen, modifizierte Saccharide. Vorstehende Saccharide können natürlich vorkommen oder synthetisch hergestellt sein.

Der Begriff "Nukleinsäure" bedeutet ein Mono-, Di- oder- Oligonukleotid. Oligonukleotide schließen auch DNA, DNA-Addukte, DNA-Konstrukte, Nukleinsäure-Analoga (z.B. PNA oder LNA), RNA (sense/antisense) oder siRNA ein.

Der Begriff "Festphasen-Oberfläche" bedeutet jegliche (modifizierte) Oberfläche, an die Biomoleküle gebunden werden können, z.B. übliche Träger für die Biochip- /Array-Herstellung, z.B. aus Glas, Folien, Membranen (PP, PE; Nylon, Cellulose, Cellulose-Mischester, PA, Halbleiter, Nanotubes, Chitin., Chitosan, semipermeable Membranen, Simplex-Membranen, Keramiken, Hybridpolymere, Nanocomposite)

Der Begriff "Nanoteilchen" bedeutet Teilchen im Größenbereich von kleiner 1000 Nanometer, die insbesondere für Beschichtungen oder diagnostische Zwecke verwendet werden. Dies sind beispielsweise "Quantum Dots" oder Gold-Nanoteilchen, die insbesondere für die elektronenmikroskopische Untersuchung von Biomolekülen herangezogen werden.

Diagnostika ist eine Sammelbezeichnung für die in der klinischen Chemie und in medizinischen Laboratorien für die biochemische Analyse eingesetzten Reagenzien, die eine medizinische Diagnose (griechisch diágno̅sis = Erkenntnis, Beurteilung) oder die Überwachung therapeutischer Maßnahmen unterstützen, indem Informationen über physiologische oder pathologische Zustände generiert werden. Die damit durchgeführten Untersuchungen können *in vivo* und *in vitro* erfolgen. Man kann bei den Diagnostika chemische, biochemische, immunologische und DNA-analytische Verfahren unterscheiden: Der Begriff "Diagnostika" beinhaltet z.B. radiochemische Verbindungen einschließlich Fluor 18 für PET-Untersuchungen, Quantum-dots, Farbstoffe und Antikörper für in vitro und in vivo Untersuchungen.

Die DARinv ist ein Standardverfahren der organischen Chemie und die Reaktionsbedingungen sind einem Fachmann wohl bekannt bzw. können in einschlägigen Lehrbüchern nachgeschaut werden. Im Rahmen der vorliegenden Erfindung wird die DARinv vorzugsweise in beliebigen Lösungsmitteln zwischen 20°C und 100°C durchgeführt. Bevorzugte Lösungsmittel sind Wasser oder Alkohole, wie Methanol oder Ethanol, Dichlormethan, Dioxan, Tetrahydrofuran aprotische polare Lösungsmittel, wie DMF.

Das vorstehend beschriebene System eignet sich auch zur kontrollierten Freisetzung von Teilen der Dienophil- oder Dien-Komponente, nach erfolgter Verankerung dieser Komponente an einem Polymerträger durch DARinv. Trägt das z.B das Dienophil ein Therapeutikum so steht damit ein System zur Verfügung, das für die kontrollierte und steuerbare Freisetzung von Arzneimitteln von einer Festphase genutzt werden kann., entweder durch hydrolytische oder enzymatische Spaltung am Wirkort In Erweiterung vorstehend beschriebener Reaktionen steht mit dem erfindungsgemäßen Verfahren eine Möglichkeit der Verknüpfung von Peptiden mit Sacchariden, von Peptiden mit Nukleinsäuren, von Sacchariden mit Nukleinsäuren und der jeweiligen Komponente mit sich selbst zur Verfügung, sofern die eine Komponente mit einem Dien und die andere mit einem Dienophil verbunden wäre. Die entstandenen Diels-Alder Addukte können selbst noch abgewandelt werden, z.B. durch Oxidation oder auch durch Hydrierung der Doppelbindung oder durch Additionsreaktionen an diese Doppelbindung.

Um größere Cluster oder Bibliotheken aufzubauen, müssen die Schritte des erfindungsgemäßen Verfahrens mehrmals hintereinander durchgeführt werden.

Erfindungsgemäß können die Dien- oder die Dienophil-Komponente mehrfach in einem Molekül vertreten sein. Das gilt auch für die bereits beschriebenen Kombinationsmöglichkeiten mit der klassischen DAR. Geeignete, leicht zugängliche Verbindungen sind unter Linkersysteme aus Dienophilen benannt. Gleiches gilt für die Diene, wobei besonders die Kombination aus Tetrazin und Triazin wegen der unterschiedlichen Dien-Reaktivität in der DARinv von besonderem Interesse ist.

Nachfolgend sollen einige wichtige bevorzugte Aspekte der vorliegenden Erfindung hervorgehoben werden, wobei diese nicht als Beschränkung des breiten Verfahrenskonzepts auszulegen sind.

Im folgenden werden daher zunächst einige Darstellungsmöglichkeiten von Dienen, dann der Dienophile und schließlich die Diels-Alder Reaktion beschrieben.

### Darstellung von substituierten Triazinen und Tetrazinen (Dienkomponente):

### Tetrazine:

Tetrazine besitzen eine hohe Reaktivität als Diene in der inversen DAR. Die Modellverbindung für viele Untersuchungen ist der gut zugängliche Tetrazin-dicarbonsäure-dimethylester. Die für die erfindungsgemäßen Zwecke notwendigen Veränderungen an dieser Verbindung, z.B. nukleophile Substitutionen, führen jedoch zur Zersetzung. Dagegen lassen sich viele Umsetzungen der Esterfunktion mit Nukleophilen problemlos und schnell an der Dihydro-Vorstufe durchführen. So gelingt es leicht monosubstituierte Verbindungen herzustellen und damit den Weg zur Darstellung unsymmetrischer Verbindungen zu eröffnen. Die nachfolgende Oxidation der Dihydro-Verbindungen zu den Tetrazinen läßt sich mit einer Vielzahl von Oxidationsmitteln, wie Fe(III)Cl₃, Nitrit, Brom oder H₂O₂ durchführen. Die aufgezeigten Verbindungen sind nachfolgend dargestellt und veranschaulichen die Möglichkeiten des Synthesekonzeptes. So lässt sich für jede Anwendung die geeignete Verbindung herstellen.

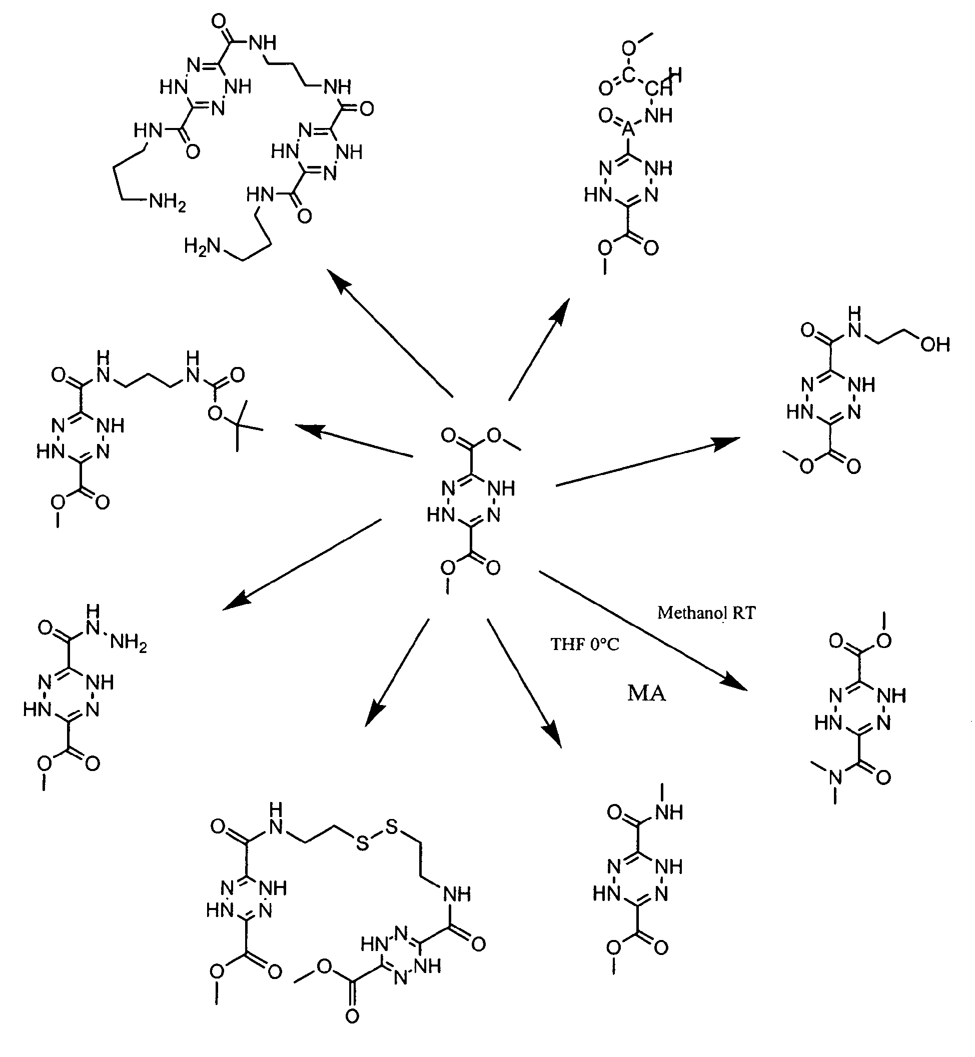

Neben den Tetrazinen mit Esterfunktionen sind viele Tetrazine mit aromatischen Resten literarurbekannt. Sie verfügen gegenüber dem Tetrazin-dicarbonsäure-dimethylester über eine verringerte Dien-Aktivität bei gleichzeitigem deutlichen Zuwachs an Stabilität. Es stehen auch Methoden zur Verfügung um unsymmetrisch substituierte Verbindungen herzustellen. Die Einführung von elektronenziehenden Substituenten in den Phenylringen führt zu einer Steigerung der Dien-Aktivität. Auch hier ist das Ziel die einfache Darstellung monofunktionaler Verbindungen zum Einbau in Peptide, Oligonukleotide oder ihre Verankerung an Oberflächen. Methoden zur Darstellung solcher Tetrazine sind literaturbekannt. Die einfachste Darstellung aromatischer unsymmetrischer Verbindungen besteht in der Umsetzung zweier Amidine oder Imidoester mit Hydrazin oder zweier Nitrile mit Schwefel und Hydrazin.

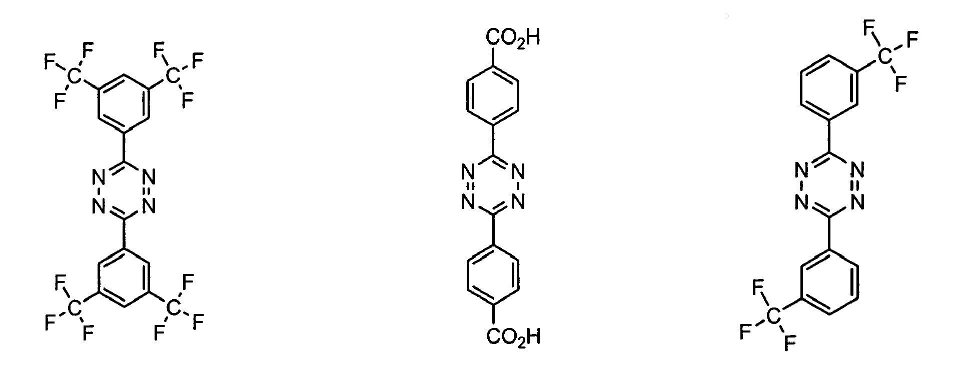

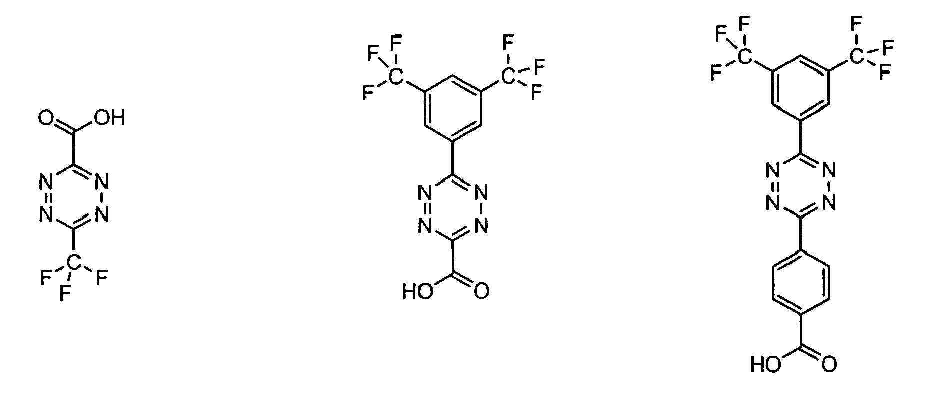

### Triazine

Im Gegensatz zu den Tetrazinen ist bei den Triazinen die Reaktivität als Dien deutlich geringer ausgeprägt. Dafür ist aber die chemische Stabilität deutlich besser und liegt in der Größenordnung normaler organischer Verbindungen. Hier hat sich als Modellverbindung der Triazin-tricabonsäure-triethylester herausgestellt. Erfindungsgemäß wird der kristalline Trimethylester bevorzugt. Nachteil der Triazine neben der geringeren Reaktivität ist die Bildung isomerer Produkte bei der DARinv. Man ist dabei mit zwei Notwendigkeiten konfrontiert: zum einen mit der Erhöhung der Reaktivität als Dien und zum anderen der Darstellung definierter monosubstituierter Verbindungen. Beides ist durch den gezielten Ersatz der Estergruppen durch Trihalogenmethylgruppen, bevorzugt Trifluormethylgruppen, zu erreichen. Nukleophile Substitution an den Estergruppen der Triazine durch Amine ist ebenso leicht durchführbar wie bei den Tetrazinen. Aufgrund der geringeren Reaktivitätsunterschiede ist dagegen eine eindeutige Monosubstitution nicht so leicht möglich. Auch hier ist das Ziel über diese nukleophile Substitution funktionelle Gruppen wie Carboxy, Hydroxy und Amino einzuführen. Über diese Funktionen können dann Reportermoleküle, Therapeutika, Peptide oder Oligonukleotide eingeführt werden.

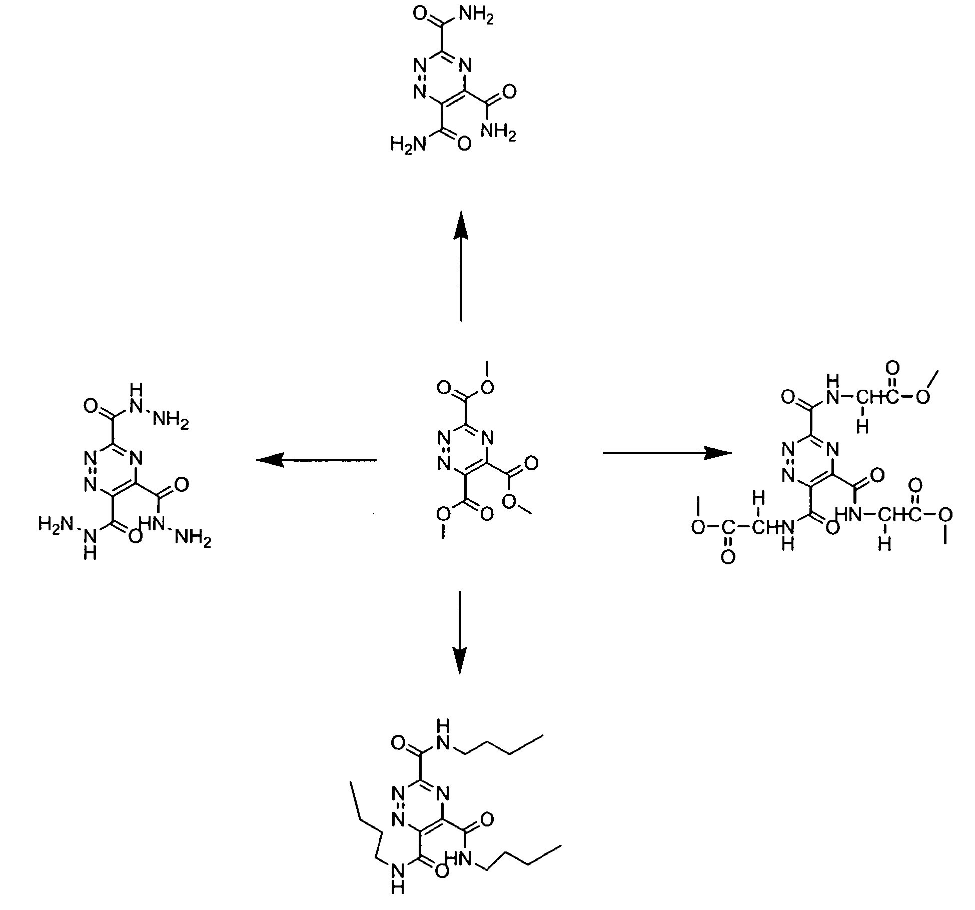

Ein bevorzugtes Triazin ist 3-Carboxymethyl-5,6-bis-trifluormethyl-Triazin 1,2,4. Dieses Triazin verfügt nur über eine Funktion, über die eine Reihe von reaktiven Gruppen eingeführt werden können, die für den angestrebten Verwendungszweck geeignet sind. Durch die beiden benachbarten Trifluormethylgruppen neigen diese Triazine zur Hydratbildung, eine Eigenschaft, die von vielen Triazinen bekannt ist. Diese Hydratbildung kann u.U. die Aktivität als Dien bei der DARinv veringern, aber die DARinv findet statt.

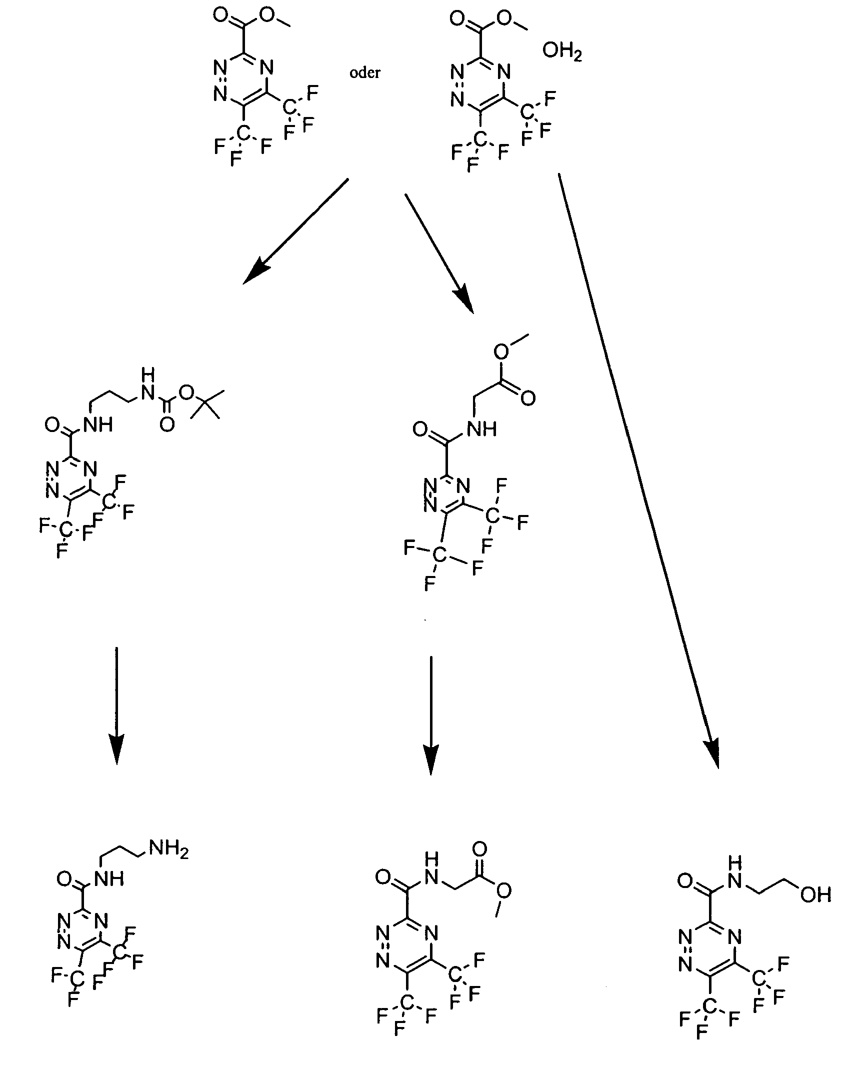

### Diazine

Durch die DARinv von Terazinen mit einem Dienophil entstehen Dihydrodiazine, aus denen durch Oxidation Diazine leicht zugänglich sind. Ist das Dienophil ein Enamin oder ein substituiertes Acetylen, so entstehen direkt die Diazine.

### Darstellung von Dienophilen

### Dienophile I

Eine Reihe bekannter cyclischer und bicyclischer ungesättigter Anhydride unterschiedlicher Ringgröße lassen sich problemlos mit Aminen zu Säureimiden umsetzen. Hier sei explizit auf das exo- und das endo-Norbornendicarbonsäureanhydrid hingewiesen, die beide käuflich sind. Das Amin lässt sich leicht weiter substituieren zu einem Dienophil, auch mit der Peptid-Technologie, welches dann an ein Tetrazin tragendes Molekül, auch an einer Oberfläche, kovalent geknüpft werden kann. Die Umsetzungen mit Lysin liefen Peptid-Bausteine, die gezielt an beliebigen Positionen in Peptiden eingebaut werden können.

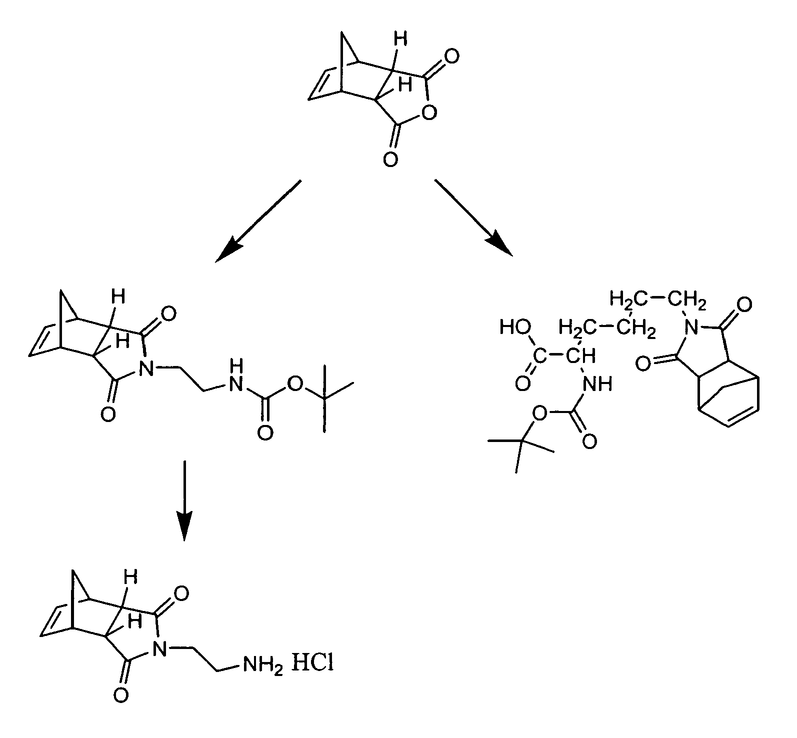

Diese Reaktionen sind übertragbar auf das exo-Norbornendicarbonsäureabhydrid, das Cyclobuten-dicarbonsäureanhydrid und das Cyclohexen-dicarbonsäureanhydrid oder beliebige andere Anhydride, die eine gespannte oder eine endständige Doppelbindung enthalten.

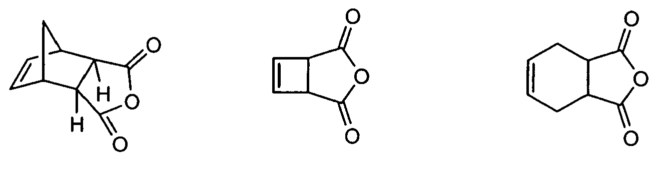

### Dienophile II

Einen besonderen Fall stellt das aus Cyclooctatetraen, kurz COT genannt, und MSA leicht zugängliche tricyclische Anhydrid dar. Durch die DAR der bicyclischen Form des COT resultiert ein Molekül, das zwei unterschiedlich reaktive Dienophile für die DARinv enthält, einen sehr reaktiven Cyclobuten-Ring und einen weniger reaktiven Cyclohexen-Ring. Damit können ganz gezielt zwei gänzlich verschiedene Moleküle in vorgegebener Weise miteinander verknüpft werden. Über den Anhydrid-Ring können in gewohnter Weise weitere Funktionen eingeführt werden, wie Aminosäuren, Amine oder die Kopplung an eine Festphase.

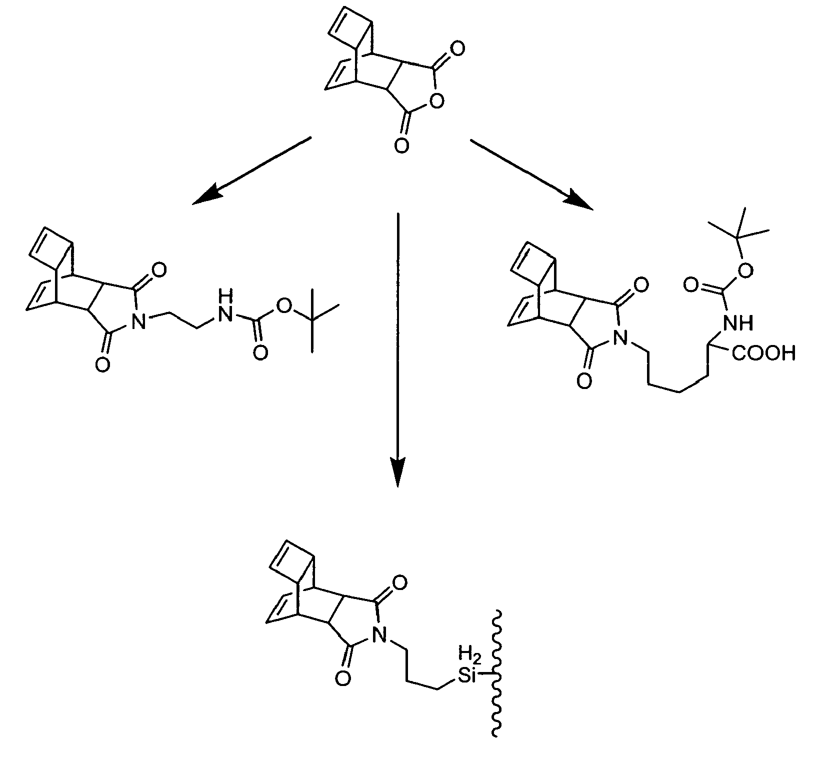

### Dienophile III Bausteine der Peptid-Synthese

Diese Aminosäuren können käuflich erworben werden und alle drei reagieren als Dienophile in der DARinv. Die drei Aminosäuren lassen sich an beliebigen Positionen während der Peptidsynthese einführen und an diesen Positionen können dann neue Reste über die DARinv eingeführt werden; auch lassen sich solche Peptide für detaillierte Strukturuntersuchungen in definierter Weise an Oberflächen verankern.

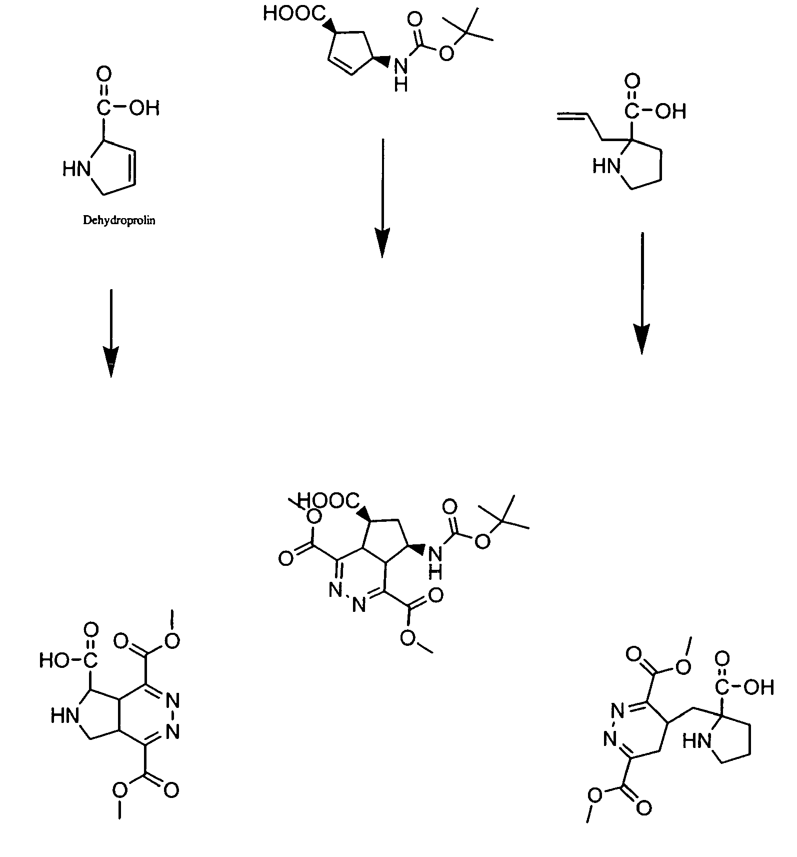

Das umgekehrte Prinzip, nämlich der Einbau des Diens in das Peptid kann auch an der Festphase durchgeführt werden. Nach der Kopplung der letzten Aminosäure, z.B. einem Glycin, wird die Schutzgruppe an deren Aminogruppe abgespalten und dann mit dem Dihydro-tetrazin-dicarbonsäureester zum Amid umgesetzt. Danach wird das Dihydrotetrazin zum Tetrazin oxidiert, z.B. mit Wasserstoffperoxid und nachfolgend mit dem Dienophil, das mit Sacchariden, Therapeutika oder Diagnostika substituiert ist, umgesetzt. Nach der Abspaltung von der Festphase und der Entfernung der Schutzgruppen kann sich, falls notwendig, die Reinigung anschließen.

### Dienophile IV

Alle Verbindungen, die eine endständige Doppelbindung besitzen, reagieren als Dienophile in der DARinv. Nachfolgend sind Umsetzungen mit käuflichen Verbindungen aufgeführt. Das sind Allyl-malonester, Allyl-Galaktose und Allyl-Silsesquioxan.

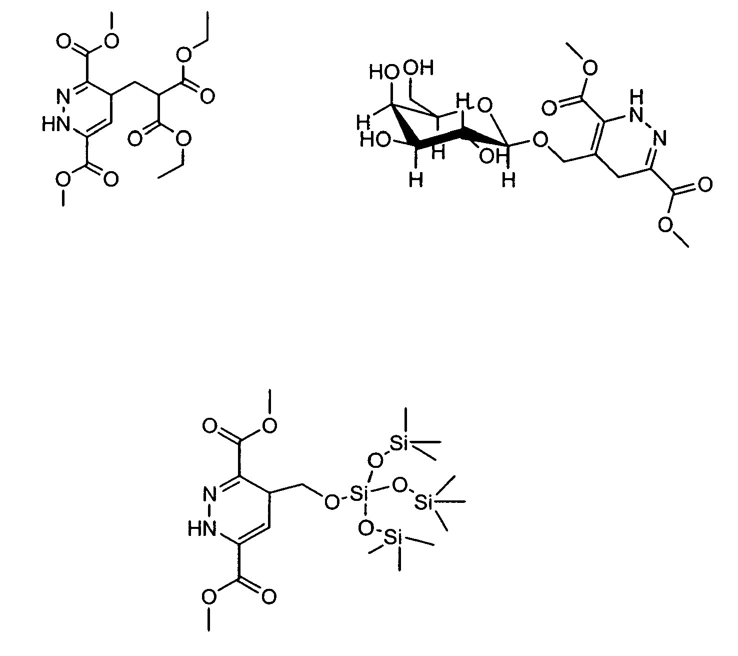

Der Anwendungsbereich der auf der DAR inv. aufbauenden Ligations-Technologie reicht von der Funktionalisierung von Oberflächen im Bereich der Nanomaterialien bis zur Markierung von Biopolymeren mit Farbstoffen oder anderen Reportermolekülen. Auch die Verknüpfung von Therapeutika mit Bioplymeren im Rahmen des Drug-Ttargeting, die Verknüpfung von Proteinen mit Sacchariden zur Verbesserung der pharmakokinetischen Parameter. Im folgenden werden einige Anwendungen detailliert beschrieben.

### Anwendungen I Platin-Komplexe

Ziel ist es Platinkomplexe herzustellen, bei denen entweder der Diaminligand oder die Abgangsgruppe als Dienophil ausgebildet sind. Dieses Vorgehen ermöglicht es, selektiv den jeweiligen Teil des Komplexes gezielt zu verändern. Damit wird der Aufbau von Bibliotheken des ursprünglichen Komplexes möglich. Die Veränderung der Abgangsgruppe erscheint besonders interessant, da diese ja während der intrazellulären Aktivierung der Pt-Komplexe abgespalten wird und damit auch der jeweilige durch die DARinv angefügte Teil. So ließe sich die Abgangsgruppe durch Moleküle verändern, z.B. peptidische Signalsequenzen, die eine bevorzugte Aufnahme dieser Komplexe in Tumorzellen vermitteln können. Die Ausprägung des Diaminliganden als Dienophil erlaubt nach der kovalenten Bindung des aktiven Platin-Komplexes an die DNA, die Lokalisation des entstandenen Adduktes durch eine DARinv mit einem Reportermolekül. Natürlich ist es auch möglich beide, sowohl den Aminliganden als auch die Abgangsgruppe, als Dienophile mit unterschiedlicher Dienophil-Aktivität einzubauen. Um sicher zu stellen, dass Pt²⁺ nicht mit olefinischen Doppelbindungen während der Synthese reagiert, wurden die hier aufgezeigten Pt-komplexe dargestellt.

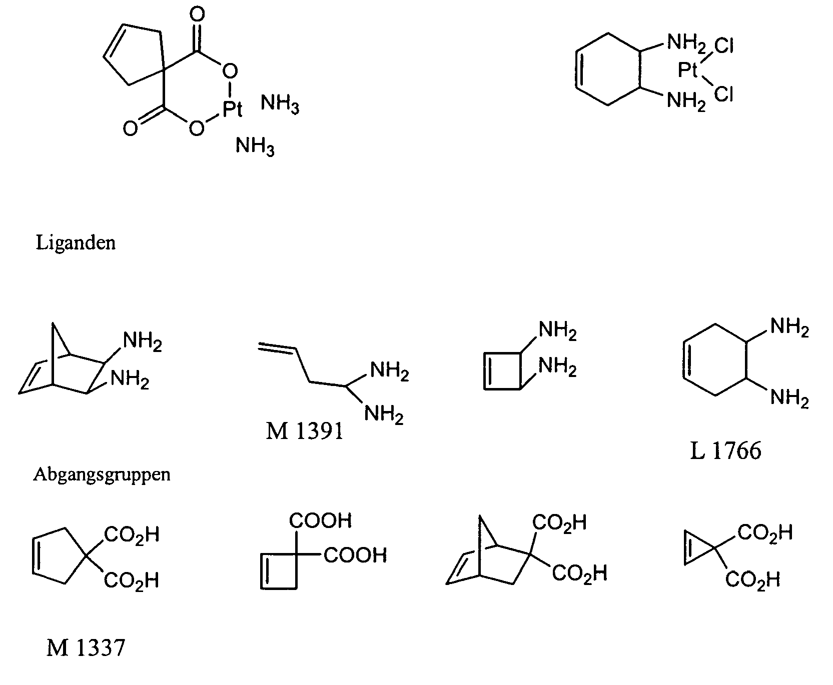

Die hier aufgeführten Liganden und Abgangsgruppen sind literaturbekannt.

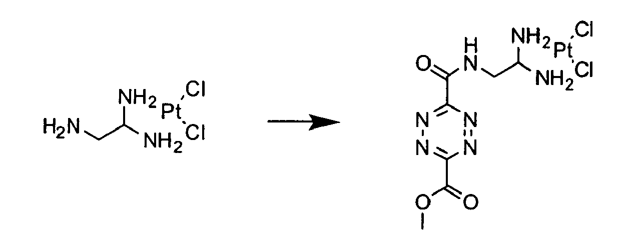

Da der gezeigte Platinkomplex des 1,2,3-Triaminopropans bekannt ist, kann über seine freie Aminogruppe an das Tetrazin gebunden werden, womit ein weiterer Baustein zum Einbau von Pt-Komplexen in Proteine, Saccharide und andere Biomoleküle und Therapeutika vorhanden ist.

### Anwendungen II PET

Die Positronen-Emissions-Tomographie stellt eine radioaktive, nichtinvasive, aber sehr empfindliche diagnostische Methode dar. Der am meisten verwendete Positronenstrahler ist F18, das mit einer Halbwertszeit von 18 min unter Abgabe eines Positrons in das Element Sauerstoff zerfällt. Wegen der geringen Halbwertszeit erfordert die Darstellung geeigneter F18-markierter Verbindungen besondere Synthese-Methoden. Sie müssen schnell ablaufen und notwendige Reinigungsverfahren müssen einfach sein. Die zur Zeit am häufigsten verwendete Verbindung ist 2-Fluor18-2-deoxy-glucose. Die Ligationsreaktion auf der Basis der DARinv läßt sich sehr gut zur Markierung von Peptiden, Oligonukleotiden und Sacchariden mit F18 verwenden. Nukleophile Substitutionsreaktionen an Aromaten werden erleichtert, wenn die Zahl der Stickstoff-Atome im Ring zunimmt, etwa in der Reihe Benzol, Pyridin, Pyrimidin und Triazin. So lässt sich im 1,2,4-Triazin ein Thiomethyl-Rest sehr leicht durch eine Reihe von Nukleophilen ersetzen, auch durch Halogene. Da solche Triazine aber als Diene in der DARinv reagieren, bietet die vorherige Einführung von F18 in ein solches Triazin die elegante Möglichkeit die oben genannten Biopolymere mit Hilfe der DARinv mit F18 zu markieren und sie somit dem Nachweis durch PET zugänglich zu machen.

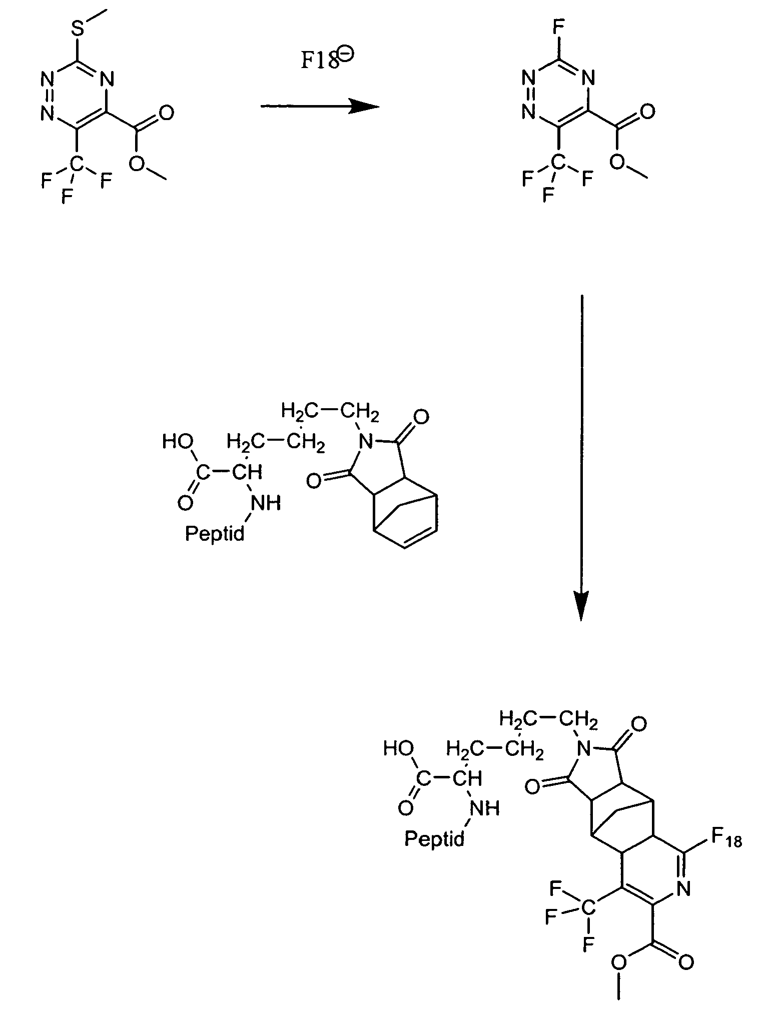

Auch die Einführung von Trifluoracetyl-gruppen, die F18 markiert sind, über die Amin-funktion der beschriebenen Tetrazine und Triazine kann hier verwendet werden.

### Anwendungen III Oberflächen

Wie bereits bei der Darstellung von Dienen beschrieben, lässt sich der Dihydro-Tetrazin-dicarbonsäureester bei RT mit primären Aminen zu Amiden umsetzen. Diese hohe Reaktionsfähigkeit kann zum Aufbau von reaktiven Festphasen herangezogen werden. Die hier gezeigten Reaktionssequenzen lassen sich in Ausbeuten zwischen 70 und 90% durchführen. Damit werden Festphasen zugänglich, die entweder ein Dien oder ein Dienophil für die DARinv tragen. Dabei ist die Reaktionsfähigkeit in der DARinv so hoch, dass die dienophiltragende Festphase mit einem Tetrazin regelrecht titriert werden kann. Die Anwendungen, die sich daraus ergeben, reichen von der Chiptechnologie für Oligonukleotide, Proteine oder Saccharide bis hin zu katalytischen Oberflächen und Festphasenreagentien.

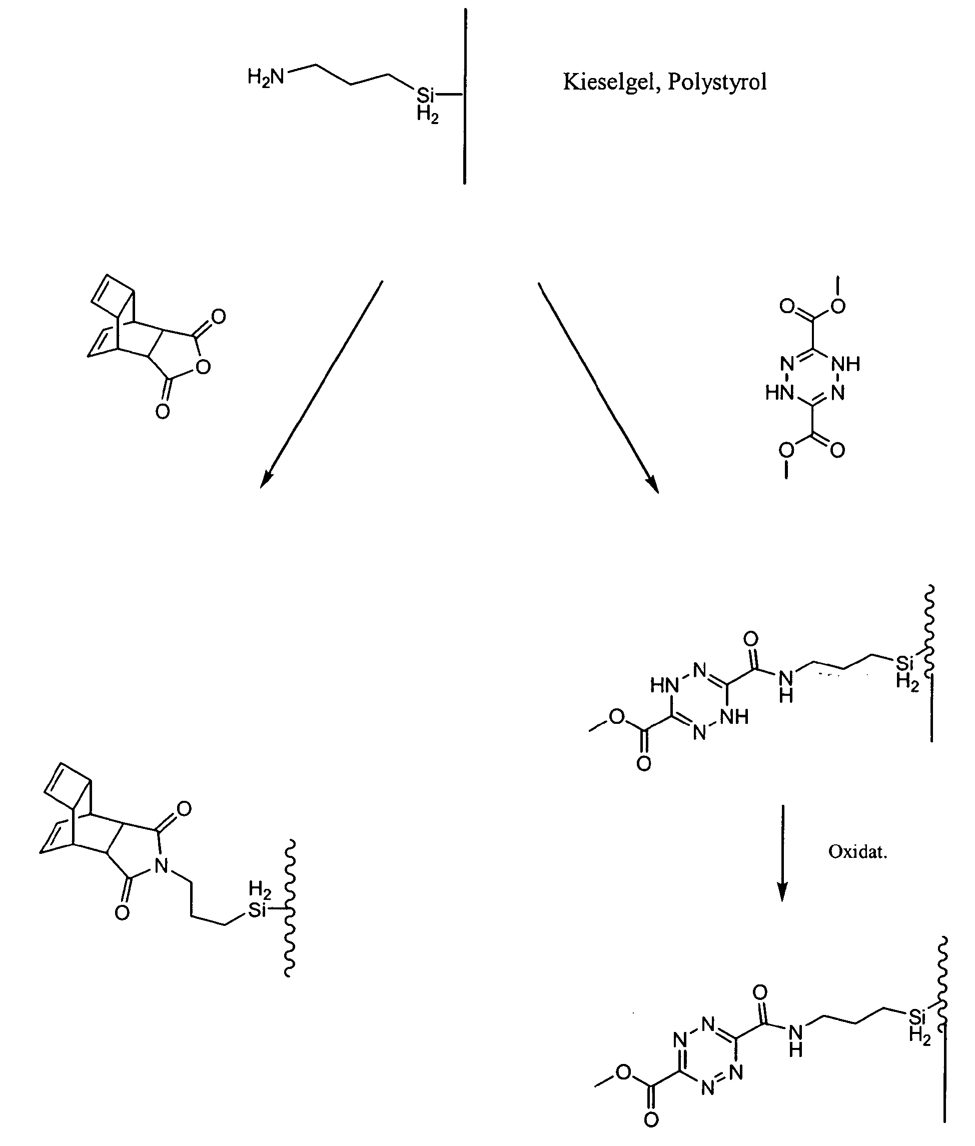

Da die DARinv eine sehr schnelle Reaktion ist, können damit auch zwei Oberflächen kovalent miteinander im Sinne eines Klebstoffes verbunden werden.

### Anwendungen IV DNA-Addukte

Die analytischen Methoden zur Detektion von DNA-Addukten sind nach wie vor nicht automatisierbar. Das P32-post-labeling Verfahren ist immer noch die empfindlichste Methode, allerdings ist bisher keine Methode verfügbar, die den gleichzeitigen Nachweis unterschiedlicher Addukt-Typen gestattet. Die Methode der Trennung mittels Kapillar-Elektrophorese mit anschließender Fluoreszenz-Detektion ist zwar zum Nachweis von 5-Methyl-Cytosin sehr gut geeignet, verfehlt aber die Nachweisgrenze des 32P-post.labeling Verfahrens um mindestens den Faktor 100. Hier kann auch die in der vorliegenden Anmeldung beschriebene Ligationsreaktion Abhilfe schaffen. Das vom Norbornen abgeleitete Amin läßt sich mit einer neueren Methode nach Literatur an die Phophatgruppe der Nukleoside koppeln. Dann kann über die DARinv ein beliebiger Fluoreszenzfarbstoff, der mit einem Tetrazin oder Triazin verknüpft ist, angekoppelt werden und anschließend mittels der Kapillarelektrophorese detektiert werden.

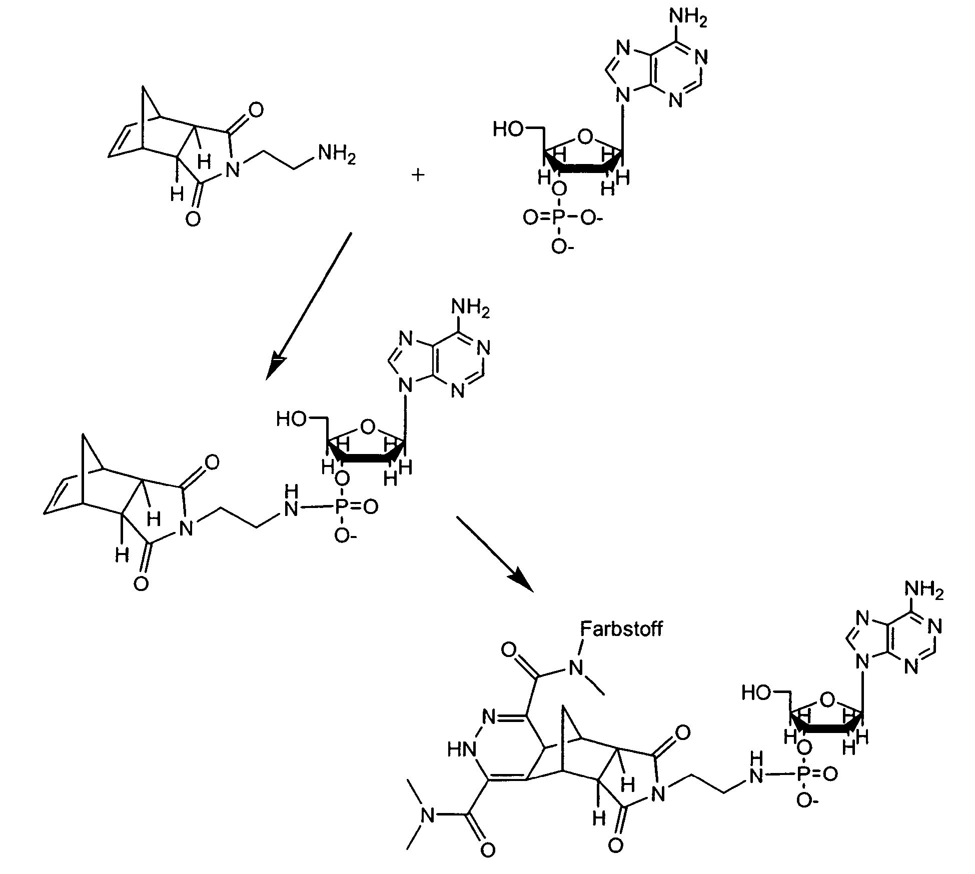

Neben diesem allgemeinen Verfahren besteht die Möglichkeit diejenigen DNA-Addukte, die strukturell eine DARinv fähige Doppelbindung enthalten, direkt nachzuweisen, so zum Beispiel die EthenoAddukte von dA und dC. Diese Addukte spielen eine wichtige Rolle bei der Beurteilung von oxidativem Stress.

Mit Hilfe der neuen Ligationsreaktion können beliebige Substituenten in durch Synthese gewonnene Oligonukleotide eingeführt werden. Dazu genügt die Einführung eines Nukleotides während der Synthese, das einen Alkylrest mit einer terminalen Doppelbindung trägt.

### Anwendungen V Photochemie

Die photochemische Cyclisierung von 1,3-Dienen zu Cyclobutenen verläuft mit hoher Quantenausbeute wobei ein sehr reaktives inverses Dienophil generiert wird. Damit kann die Photolithographie mit der Ligation durch die DARinv verknüpft werden. Denn nur dort wo UV-Licht eingestrahlt wird, findet die 2+2 Cycloaddition statt und nur dort kann anschließend die DARinv ablaufen. So lassen sich auch hier substituierte Tetrazine und Triazine an Oberflächen addieren.

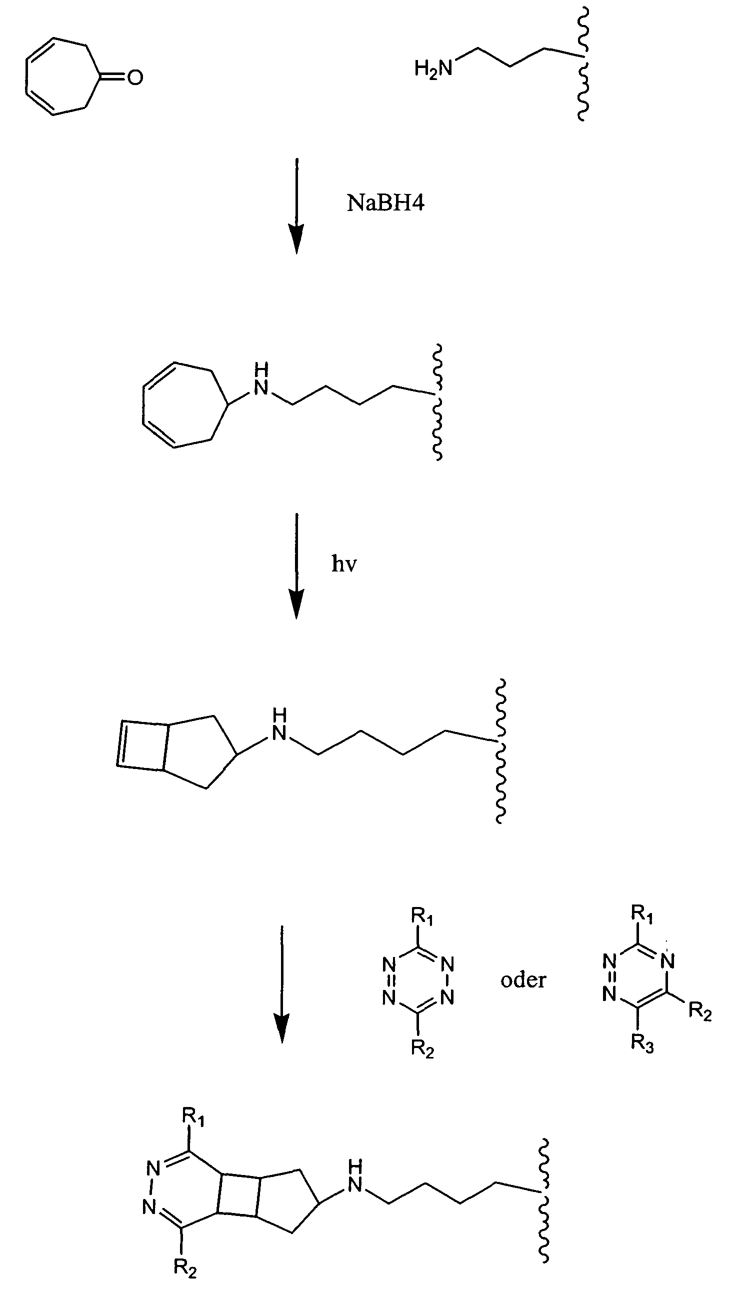

Auch die Einführung des 1,3-Dihydro-phthalsäureanhydrides-5,6 durch die Reaktion mit der Amino-funktion ist hier geeignet, Auch die bisher nicht bekannte 1,3-Cycloheptadien-6-carbonsäure ist hier geeignet.

### Anwendungen VI Aufbau von Dendrimeren

Die Reaktion des COT-MSA Anhydrides ist so schnell und eindeutig, dass sich durch Umsetzungen mit Polyaminen dendritische Strukturen aufbauen lassen, die nun durch Umsetzung mit beliebigen Dienen durch die DARinv weiter modifiziert werden können. Die hier gezeigten Verbindungen sind dargestellt und charakterisiert. Sie erlauben die Einführung von zwei verschiedenen Molekülen pro Bicyclus, da der Vierring und der Sechsring deutlich unterschiedliche Dienophil- Aktivitäten besitzen. Aber auch andere polyfunktionelle Moleküle, wie z.B.lnositol, lässt sich leicht mit Allylgruppen versehen und damit als Templat für dendritische Strukturen verwenden. Zu diesen Verbindungen gehört auch das Chitosan.

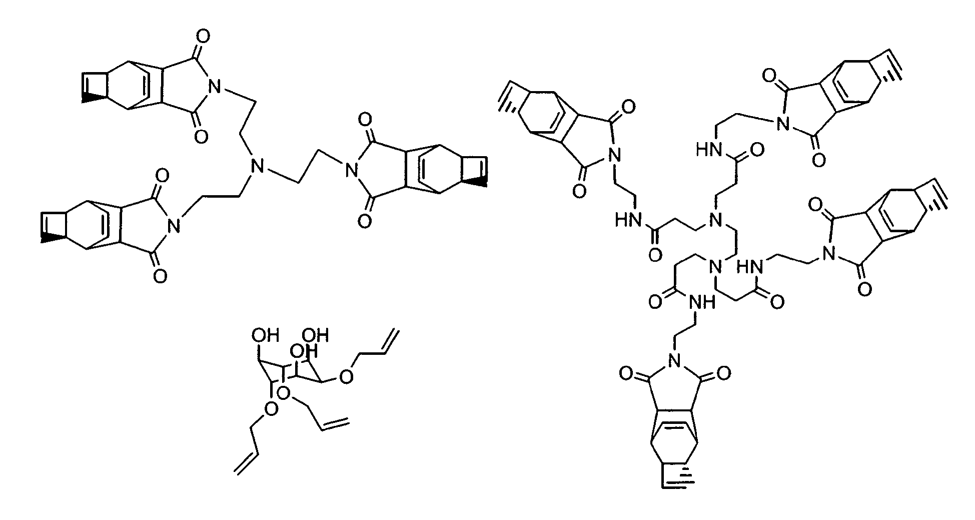

Mit einer solchen Technologie lassen sich beispielsweise Peptide oder Saccharide zur Verwendung als Therapeutika, Diagnostika oder auch zur Untersuchung der Interaktion von Peptiden oder Sacchariden untereinander oder mit anderen Biomolekülen bündeln. Die Dihydro-tetrazindicarbonsäure kann auch in Polyamide während der Kondensation eingebaut werden und nach Oxidation zum Tetrazin durch DARinv modifiziert werden. Oligomere Tetrazine oder gemischte oligomere Tetrazine/Triazine lassen sich mit den bereits vorstehend dargestellten Bausteinen herstellen und dann selektiv durch DARinv modifizieren. Auch hier sind die entsprechenden Polymeren denkbar.

### Anwendungen VII Quantum Dots

Unter Quantum Dots versteht man Nanoteilchen, die aus Verbindungen wie CdS oder CdSe aufgebaut sind und über besondere optische Eigenschaften verfügen. Unter Anregung mit Lasern fluoreszieren sie sehr stark in Abhängigkeit von ihrer Größe und finden daher immer mehr Verwendung im diagnostischen Bereich, zumal sie den Nachweis von Einzelmolekülen möglich machen. Voraussetzung dafür ist aber ihre Dotierung mit funktionellen Gruppen, die über SH-Gruppen verläuft und eine anschließende Interaktion mit den zu detektierenden Molekülen gestatten. Gold-Nanoteilchen werden aufgrund ihrer besonderen Eigenschaften für elektronenmikroskopische Untersuchungen von Biomolekülen herangezogen. Auch hier wird die Verankerung von Molekülen an der Oberfläche über SH-Gruppen bewerkstelligt. Auch hier lässt sich die neue Ligationstechnik durch DARinv einsetzen. Zu diesem Zweck wurden SH-Gruppen-haltige Triazine und Tetrazine hergestellt, wobei zunächst die Disulfide hergestellt wurden und daraus durch Reduktion mit Dithiothreitol die Mercaptoverbindung. Ganz analog lassen sich auch SH-gruppenhaltige Dienophile vom Typ Norbornen herstellen.

Damit lassen sich Tetrazine, Triazine und Diazine über die SH-Gruppe als Diene an die Oberfäche der Quantum-Dots anlagern und sind damit der DARinv zugänglich. So können z.B. Antikörper, Peptide, Saccharide oder Therapeutika an der Oberfläche der Quantum Dots zu diagnostischen oder therapeutischen Zwecken verankert werden..

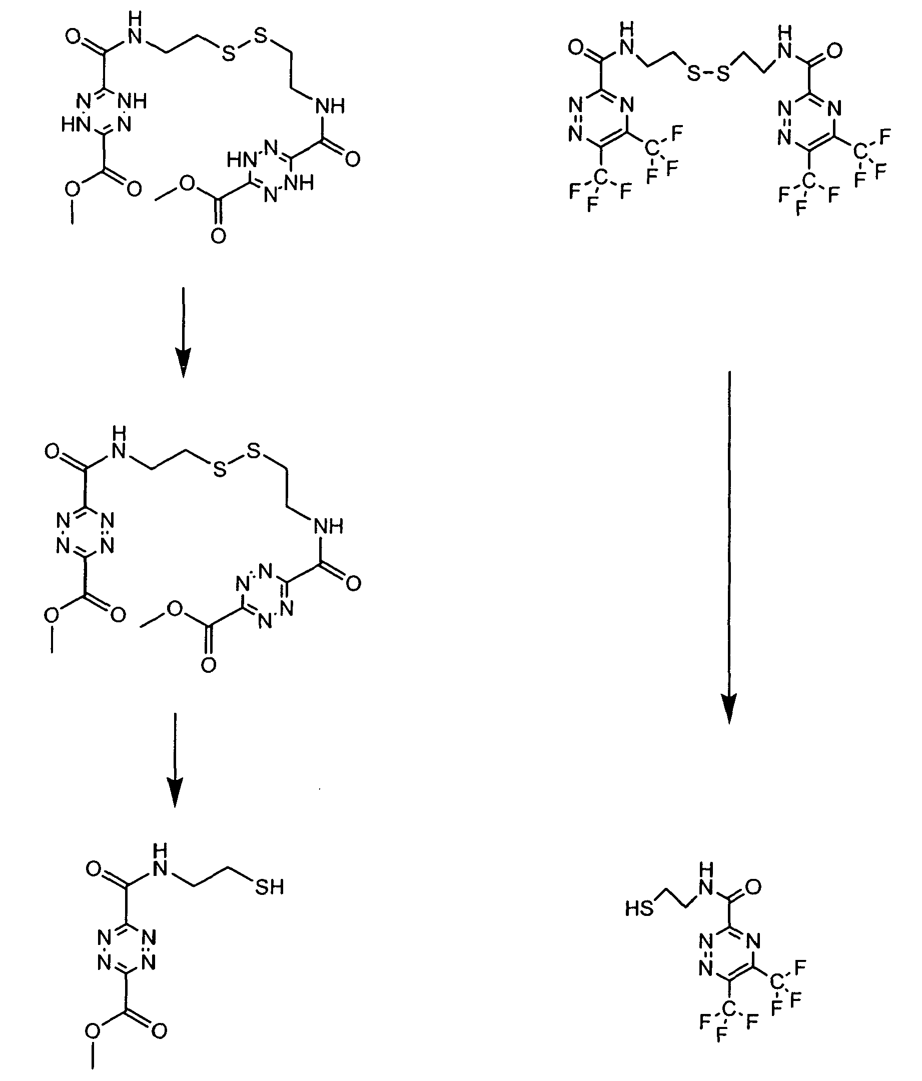

### Anwendungen VIII Saccharide

Die Synthese komplexer Saccharidstrukturen erfordert eine ausgefeilte Schutzgruppenstrategie. Dabei wird das reduzierende Ende oftmals durch eine Allylgruppe oder eine Pentenoylgruppe geschützt. Aus natürlichen Quellen isolierte Oligosaccharide können am reduzierenden Ende leicht mit einer Allylgruppe oder Pentenoylgruppe versehen werden. Damit sind die Voraussetzungen für eine Verankerung dieser Oligosaccharide an einer Festphase oder Oberfäche durch die DARinv gegeben. Die in der DE-A-100 41 221.1 beschriebenen Furan-Saccharidmimetika lassen sich ebenfalls mit Hilfe dieser DARinv Technologie entweder an Biomoleküle oder Oberflächen verankern. Voraussetzung ist die Einführung einer Allylether-Gruppe oder die Verwendung von Linkermolekülen wie sie vorstehend beschrieben sind.

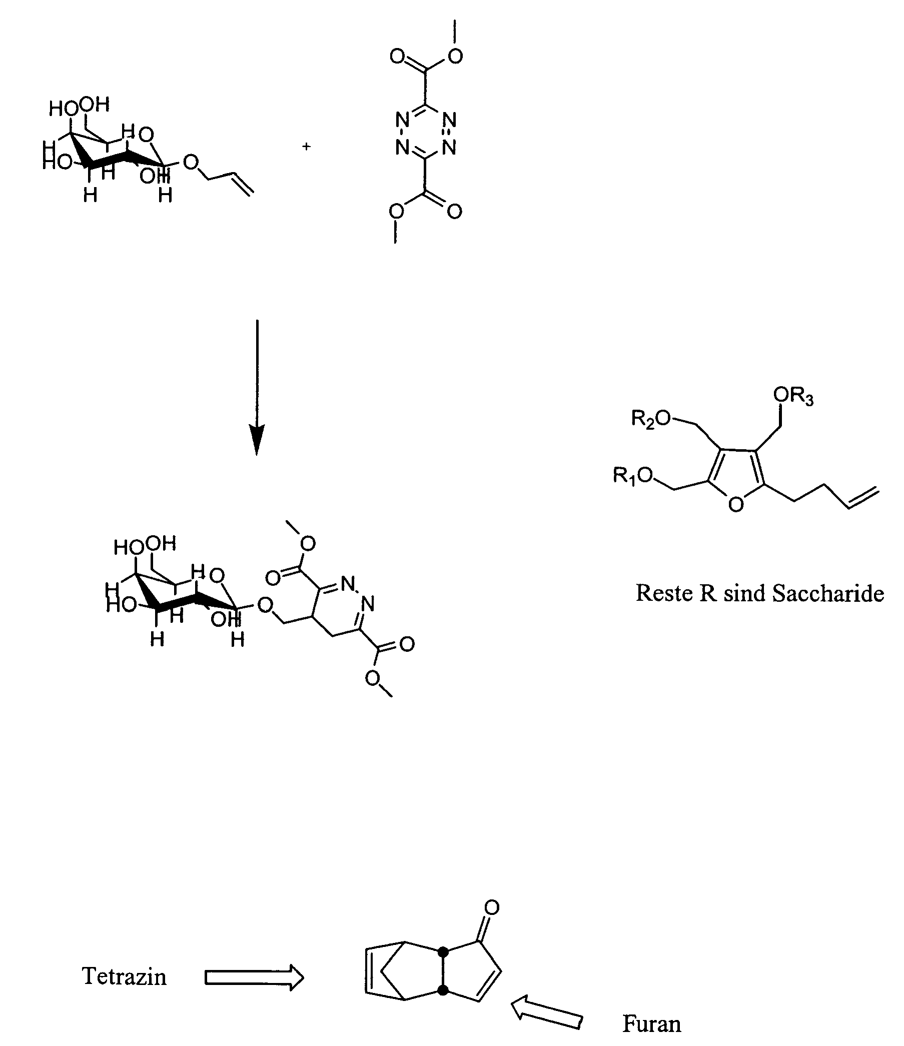

Auf diesem Wege können die Verbindungen, die in der DE-A-100 41 221.1 beschrieben sind, in der hier beschriebenen Anwendung verwendet werden.

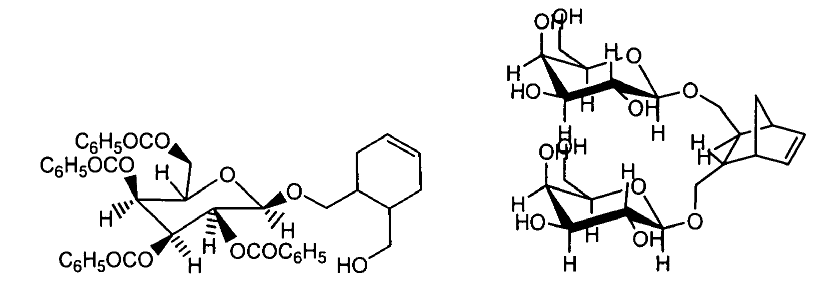

Auch diese Saccharid-Mimetika eignen sich als inverse Dienophile in der DARinv und können dadurch in beliebige Biomoleküle eingeführt werden.

### Anwendungen IX Therapeutika

Die Nebenwirkungen der Arzneimittel-Therapie, insbesondere Tumortherapie, gehören nach wie vor zum klinischen Alltag. Das liegt zum Teil daran, daß es bisher nicht gelungen ist, therapeutisch aktive Substanzen gezielt in die erkrankten Zellen einzuschleusen. Es besteht daher ein Bedarf an einfachen Ligationsreaktionen, die es gestatten, Therapeutika mit Molekülen zu verknüpfen, die eine bevorzugte Aufnahme in die Zelle ermöglichen. Bezüglich der Tumortherapie wird auf die EP-A-1 051 421 hingewiesen, in dem der Allylether eines Bor-haltigen Tetracarborans hergestellt wird, der sich nun als ideales Dienophil zur Verknüpfung mittels der DARinv an Peptide oder Saccharide erwiesen hat. Mit dieser Methode können aber auch Vitamin A, Vitamin C , Curcumin oder andere Therapeutika an Proteine oder Oberflächen gekoppelt werden und dort durch Hydrolyse oder enzymatische Spaltung freigesetzt werden. Auch Liposomen, die Doppelbindungen enthalten, die in der DARinv aktiv sind, lassen sich mit dieser Technologie sehr leicht modifizieren, um dadurch ein besseres Targeting oder eine veränderte Pharmakokinetik zu erreichen.

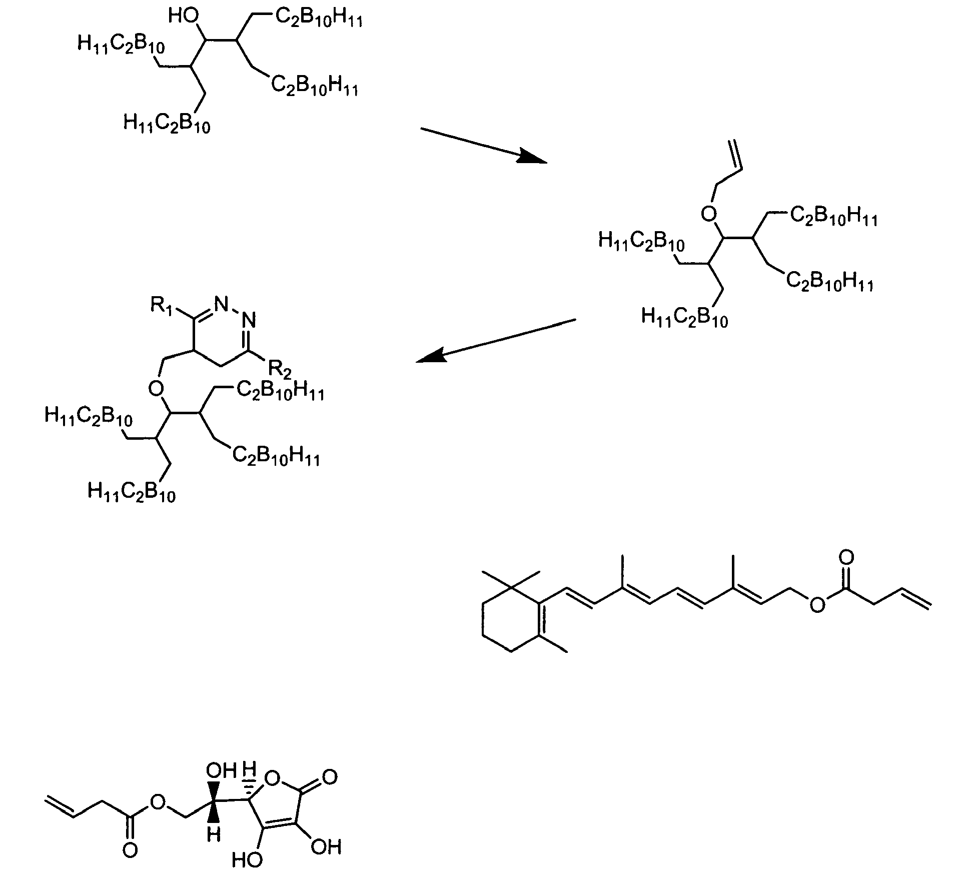

### Anwendungen X Oligonukleotide

Die bisher in der Literatur bekannten Ligationsreaktionen von Oligonukleotiden mit Hilfe der DAR benutzen cyclische Diene und als Dienophil Maleinimide.(Tona R. and Häner Robert, 2005, Bioconjugate Chem 16, 837-842; Hill K.W. et al. 2001, JOC, 66, 5352-5358). In diesen Systemen ist allerdings die Reaktionsgeschwindigkeit nicht sehr hoch und beträgt bis zu 7 Tagen, oder es müssen hohe Überschüsse an Reagentien eingesetzt werden. Hier bietet sich die DARinv als bessere, weil effizientere Ligationsreaktion an. Zu diesem Zweck wurden die beiden Amidite hergestellt um damit am Ende der Oligo-Synthese eine Allyl- oder eine Pentenylgruppe am 5'-Ende einzuführen, die in der DARinv als Dienophil aktiv ist.

| | |
|---|---|
| 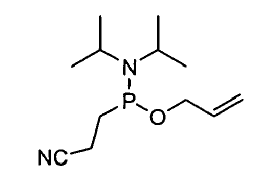 | C₁₂H₂₃N₂OP |
| | Mol. Wt.: 242,3 |
| | m/e: 242,15 (100,0%), 243,16 (13,3%), 244,16 (1,1%) |
| | C, 59.48; H, 9.57; N, 11.56; O, 6.60; P, 12.78 |
| 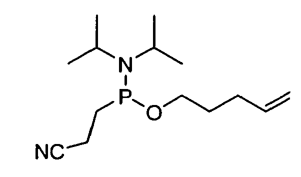 | C₁₄H₂₇N₂OP |
| | Mol. Wt.: 270,35 |
| | m/e: 270,19 (100,0%), 271,19 (15,5%), 272,19 (1,4%) |
| | C, 62.20; H, 10.07; N, 10.36; O, 5.92; P, 11.46 |

### Linkersysteme aus Dienophilen I

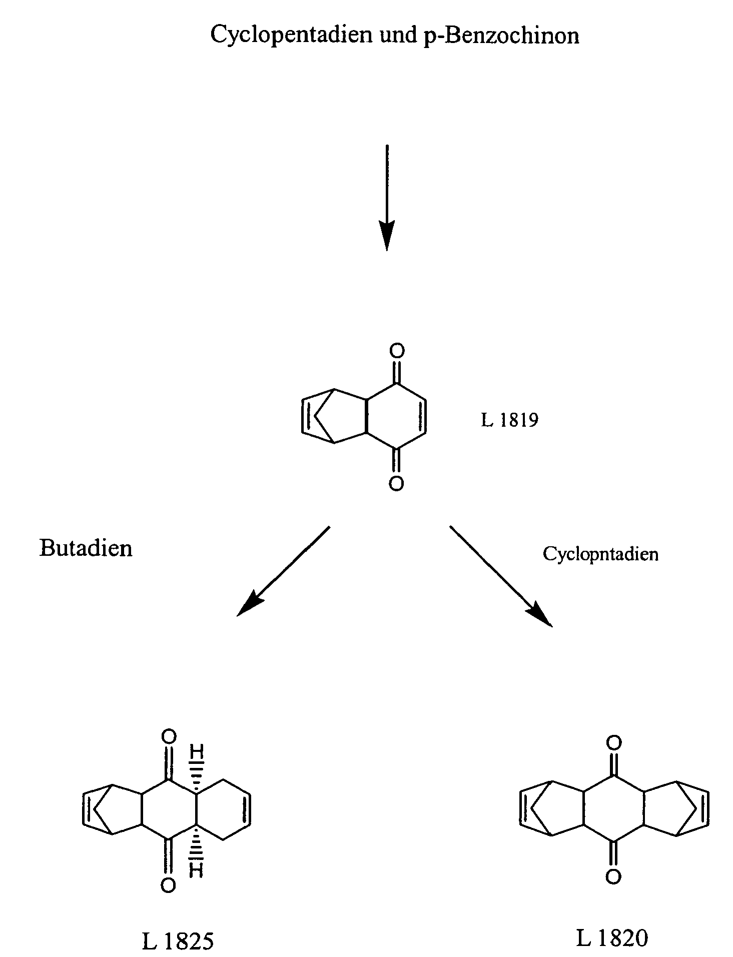

Beide Systeme sind geeignet zur aufeinanderfolgenden Verknüpfung von Molekülen, die entweder das gleiche Dienophil tragen wie L 1820 oder unterschiedliche Dienophile wie L 1825. Dieser Verbindungstyp eignet sich auch gleichzeitig für die Verankerung an einer Festphase durch Reaktion mit den oder der Carbonylgruppe

### Linkersysteme aus Dienophilen II

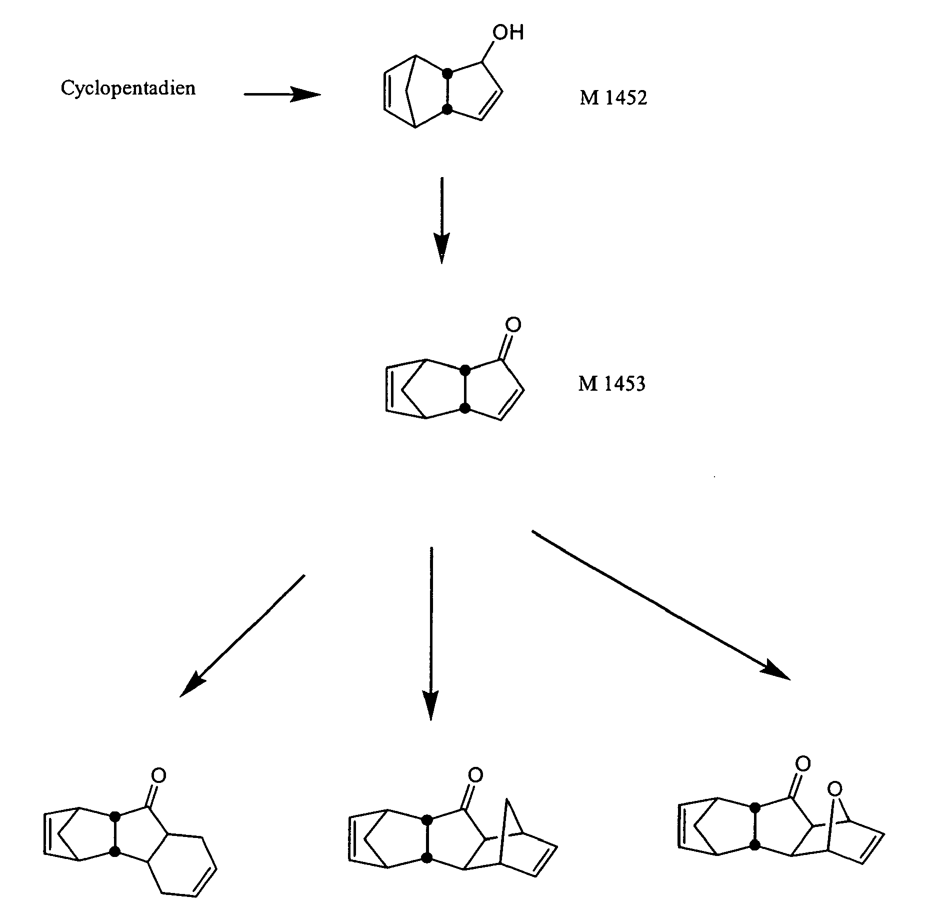

Das Keton Dicyclopentadienon ist ein orthogonales Dienophil, das sowohl der normalen DAR als auch der DARinv zugänglich ist. Durch Umsetzung mit Butadien kann es aber auch in ein doppelt inverses Dienophil überführt werden.

Neben diesen Linkern aus Dienophilen sind auch Linker aus Dienen zugänglich, die gleichzeitig eine DAR und eine DARinv zulassen. Auch die Kombination Tetrazin oder Triazin mit einem Dienophil, z.B. einem Maleinimid, für die klassische DAR ist möglich. Die Strukturen sind folgend aufgeführt.

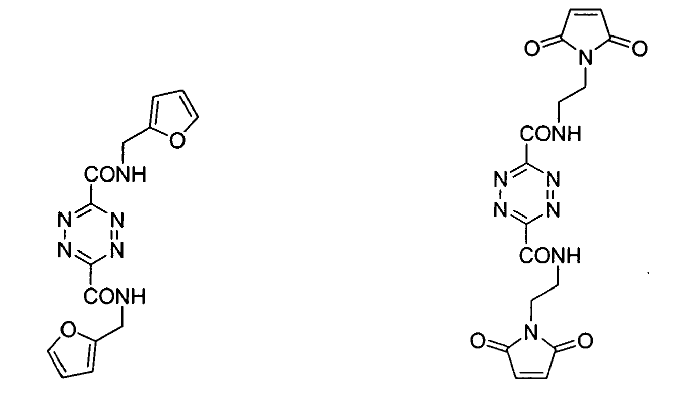

### Anwendungen XI Substanzbibliotheken

Unter Verwendung der vorliegend beschriebenen. neuen Tetrazine, Triazine und Diazine als Diene in der DARinv lassen sich mit Dienophilen, z.B. Enamine, Enolether usw., neuartige Substanzbibliotheken zur Suche nach neuen Wirkstoffen aufbauen. Für die Entwicklung neuer Therapeutika ist die ausreichende Wasserlöslichkeit. eine wichtige Voraussetzung. Ein möglicher Ansatz zur Verbesserung der Wasserlöslichkeit und damit der oralen Verfügbarkeit, ist die kovalente Verknüpfung von Wirkstoffen mit Sacchariden unter Bildung von Konjugaten. Mit dem erfindungsgemäßen Ansatz ist es möglich, unter Anwendung eines kombinatorischen Ansatzes von vornherein das Problem einer ausreichenden Wasserlöslichkeit durch die Verwendung geeigneter Bausteine in der DARinv zu berücksichtigen. Dies bietet darüber hinaus noch den Vorteil, daß diese Bausteine auf der Basis von Sacchariden und ihrer Derivate Teil des Wirkstoffs werden und damit zur Stärkung der Bindung des Wirkstoffs an sein Target beitragen können.

Die Erfindung wird weiter anhand der nachfolgenden Figuren beschrieben.
Fig. 1: Diels-Alder-Reaktion
Fig. 2: Diels-Alder-Reaktion mit inversem Elektronenbedarf

Die Erfindung wird weiter anhand der Beispiele beschrieben.

### Beispiel 1

Synthese von 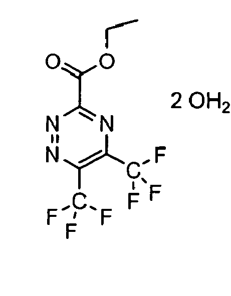 5,6-Bis-trifluormethyl-1,2,4-Triazin-3-carbonsäure-ethylester 5 Gramm (25,7 mmol) Hexafluorbuten wurden, unter Argon, in 100ml DMF gelöst, in einem 500ml Rundkolben vorgelegt und das Oxalamidhydrazon in 100ml DMF unter Kühlung bei 0°C langsam zugetropft (exotherme Reaktion !). Nach Beendigung der Zugabe wurde der Ansatz über Nacht bei Raumtemperatur weiter gerührt. Zur Aufarbeitung wurde das DMF im Hochvakuum bei 50°C abgezogen und der verbleibende Rückstand in Essigester aufgenommen und mit verd Salzsäure und Wasser gewaschen und über Natriumsulfat getrocknet. Nachdem der Essigester abgedampft war, wurde aus Di-isopropylether umkrisallisiert. Nach der gleichen Vorschrift konnte auch der Methylester erhalten werden. Beide Verbindungen werden als Dihydrate erhalten, wobei ein Molekül Wasser leicht abgegeben wird, so dass das Monohydrat ebenfalls kristallin erhalten werden kann. Massenspektrum und 1 H-NMR belegen die Strukturen.

In ähnlicher Weise konnten auch die folgenden Triazene erhalten werden

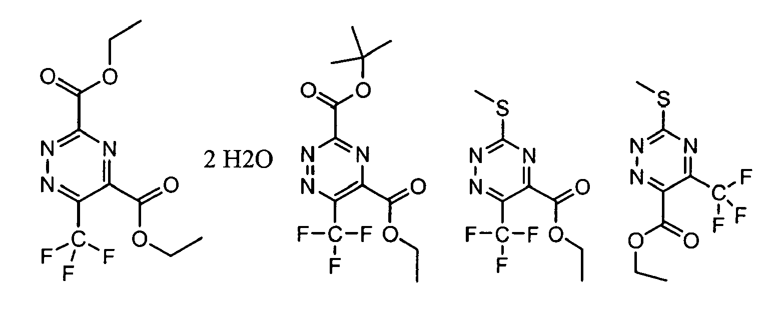

### Beispiel 2:

Synthese von 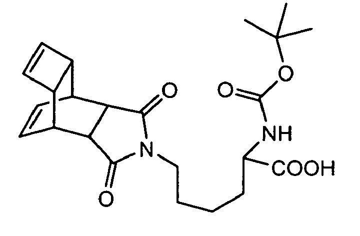 10 mmol des tricyclischen Anhydrides , erhalten aus Cyclooctatetraen und Maleinsäureanhydrid, wurden in 50ml Methanol mit 10 mmol Boc-Lysin für 3 Std. unter Rückfluss erhitzt. Der nach dem Abdampfen des Lösungsmittels verbleibende Rückstand konnte direkt umkristallisiert werden. Falls die Kristallisation nicht gelingt, kann zur Reinigung an Kieselgel chromatographiert werden, Chloroform mit 1 % Methanol. Das Produkt kristallisiert beim Stehen. Massenspektrum und NMR belegen die Struktur.

Die folgenden Verbindungen wurden analog dargestellt und charakterisiert.

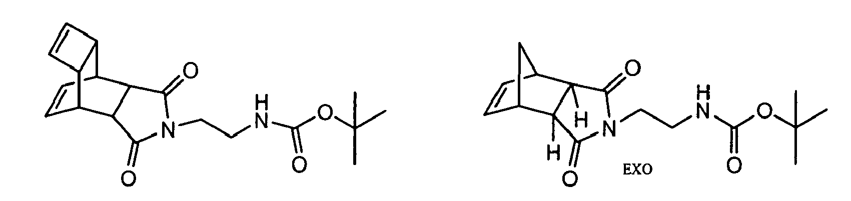

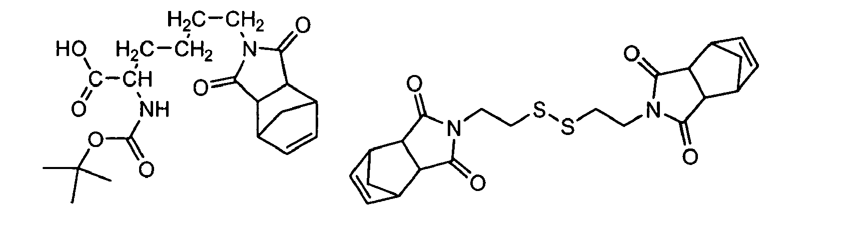

Synthese von 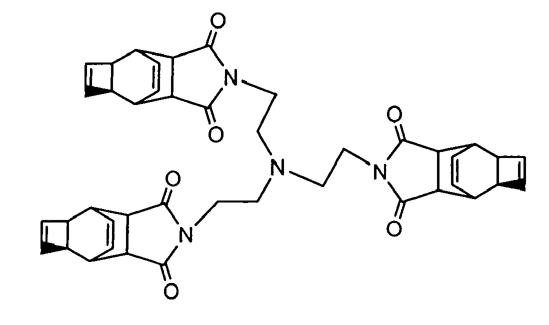 1 mmol Tris-(2-ethylamino)-amin wurden in 20 ml Methanol mit 3mmol des tricyclischen Anhydrides 5 Std. am Rückfluss gekocht. Beim Abkühlen bildet sich ein Niederschlag, der abgesaugt wird. Ausbeute 80%. Das Massenspektrum zeigt den Molekülpeak bei m/e 698 ohne nennenswerte Fragmentierung.

In analoger Weise wurde die folgende Verbindung hergestellt

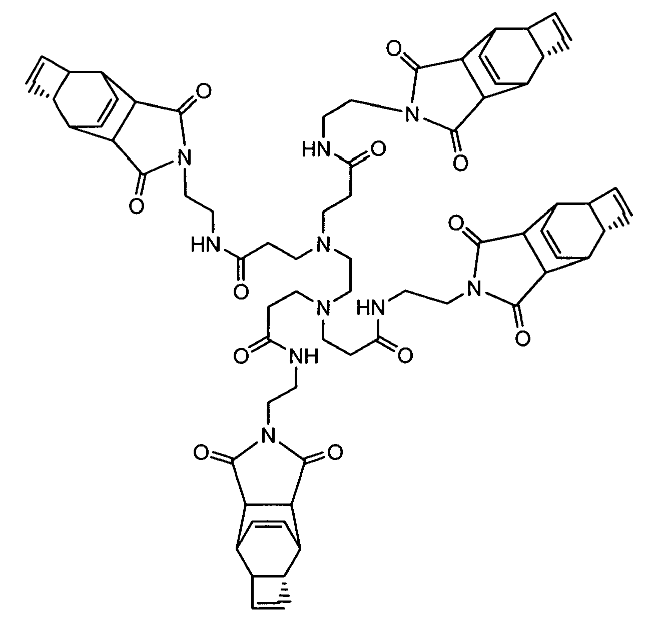

Synthese von 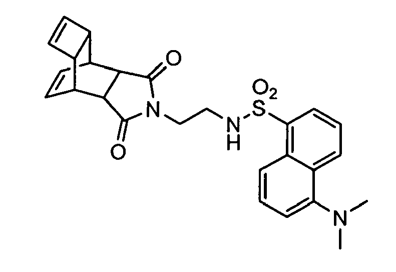 4 mmol (1,36 gr.) des durch Umsetzung des tricyclischem Anhydrides mit N-Bocethylendiamin erhaltene Amid vom Fp. 135 °C wird in einer Mischung aus 10 ml Methanol und 20ml 1 N Salzsäure über Nacht bei Raumtemperatur gerührt, wobei die Substanz in Lösung geht. Nach der Lyophilisierung wird das Hydrochlorid als weißer Rückstand in quantitativer Ausbeute erhalten. Das Massenspektrum zeigt den Molekülpeak bei 244 für das Amin.

0,5 mmol dieses Hydrochlorides wurden in 10 ml Chloroform suspendiert und dann 0,5 mmol Dansylchlorid in fester Form zugefügt. Unter Kühlung bei 0°C wurden dann 0,28 ml Triethylamin, entsprechend 2 mmol, in 5 ml Chloroform zugetropft. Der Ansatz wurde über Nacht bei Raumtemperatur gerührt. Zur Aufarbeitung wurde die organische Phase 3x mit je 10 ml Wasser gewaschen, über Natriumsulfat getrocknet und eingeengt. Zur Reinigung wurde an Kieselgel mit Hexan/Essigsäureethylester 2:1 chromatographiert. Es wurden 240 mg festes Produkt isoliert, entsp. einer Ausbeute von 50%. Das Massenspektrum zeigt den Molekülpeak bei 477 sowohl im pos. als auch im neg. Modus.

### Dienophil modifizierte Peptide:

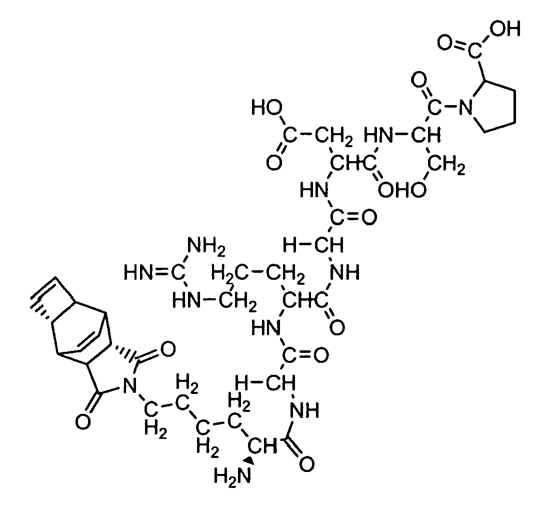

Das Peptid wurde am Synthesizer hergestellt, wobei in der letzten Stufe das bereits beschriebene Lysin-Derivat mit dem tricyclischen Dienophil angefügt wurde. Dieses Peptid wurde per HPLC gereinigt und die Struktur durch das Massenspektrum bestätigt. Molekülpeak bei m/e 899.

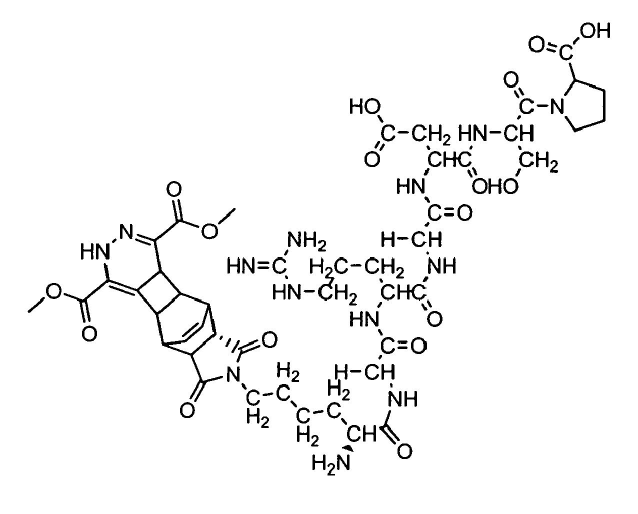

Diels- Alder-Reaktion des vorgenannten Peptides mit dem Tetrazin-3,6-dicarbonsäure-dimethylester:

0,01 mmol (9 mg) des Peptides werden in 0,5 ml DMF gelöst und mit einer 0,01 molaren Lösung des Tetrazins tropfenweise versetzt. Nach jeder Zugabe verschwindet die rote Farbe des Tetrazins sofort und es wird so lange Tetrazin zugegeben bis die rote Farbe gerade noch verschwindet. Das DMF wird im Hochvakuum verdampft. Die Masse des Rückstandes zeigt den Molekülpeak bei m/e 1096, neben einer kleinen Menge des nicht abreagierten Peptides bei m/e 899.

### Beispiel 3

### Umsetzung des Dihydro-tetrazin-3,6-dicarbonsäure-dimethylesters mit primären Aminen

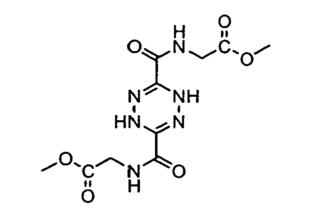

Dihydro-tetrazin-3,6-dicarbonsäure-dimethylester 200mg(1 mmol) werden in 5ml Methanol suspendiert und (2,5 mmol) Glycinmethylester in 5ml Methanol - aus 2,5 mmol Glycinmethylester-Hydrochlorid und 2,5 mmol Triethylamin zubereitet - zugetropft und über Nacht bei Raumtemperatur gerührt. Die rötliche Lösung ist hellgelb geworden und es hat sich ein gelber Niederschlag gebildet. Nach Kühlung auf -18°C wird dieser Niederschlag abgesaugt und aus Methanol umkristallisiert. Ausbeute 55%. Massenspektrum und NMR bestätigen die Struktur.

In ähnlicher Weise wurden die folgenden Verbindungen hergestellt und charakterisiert:

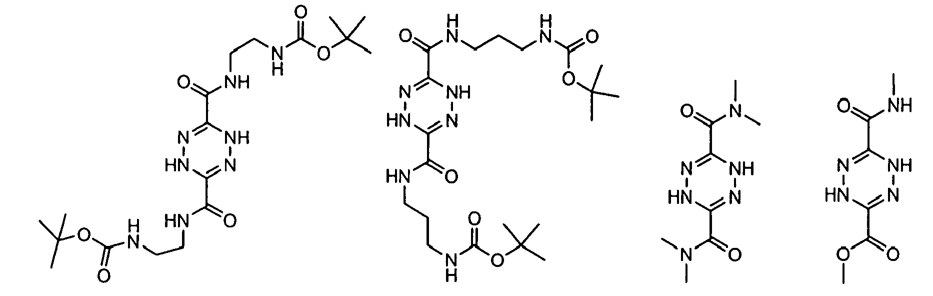

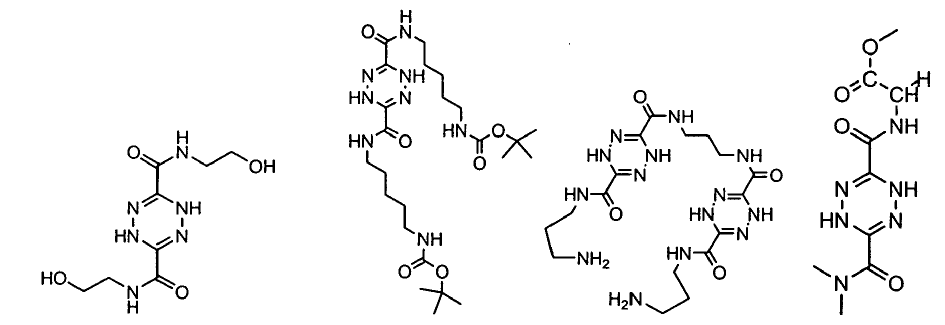

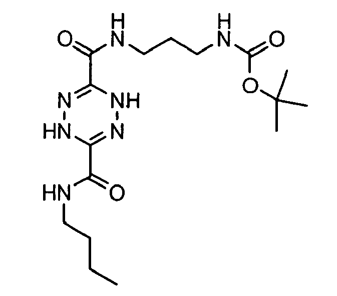

### Oxidation des Dihydrotetrazins zum Tetrazin

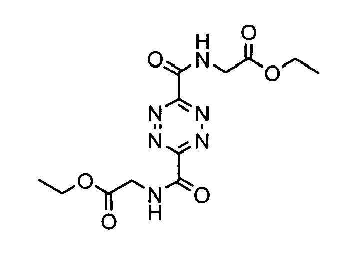

1 mmol (342 mg) des Dihydrotetrazins werden in 10ml Chloroform gelöst und mit einem kleinen Überschuß Isoamylnitrit versetzt. Die Lösung färbt sich intensiv rot und nach 2 Std. wird das Chloroform am Rot. Abgezogen und der rote Rückstand aus Aceton umkristallisiert. Massenspektrum und NMR bestätigen die Struktur des Tetrazins.

### Beispiel 4

### Umsetzung von Dihydro-tetrazin-3,6-dicarbonsäure-dimethylester mit Glycinmethylester im Verhältnis 1:1

Dihydro-tetrazin-3,6-dicarbonsäure-dimethylester 200mg (1mmol) wird in 10ml Methanol suspendiert und bei 0°C 1 mmol Glycinmethylester als Hydrochlorid in fester Form zugegeben Dann wird 1 mmol Triethylamin in 10ml Methanol langsam zugetropft. Man lässt noch 3 Std. bei dieser Temperatur rühren, dann wird die Kühlung entfernt und über Nacht unter Rühren auf Raumtemperatur kommen lassen. Die Farbe der Lösung ist nur noch hellrot und es hat sich ein gelber Niederschlag gebildet. Dieser wird nach Abkühlen auf -20°C abgesaugt, mit kaltem Methanol und mit Ether gewaschen und getrocknet. Zur weiteren Reinigung wird aus Methanol umkristallisiert. Massenspektrum und 1 H-NMR belegen die Struktur eindeutig.

Die folgenden Verbindungen wurden in analoger Weise hergestellt

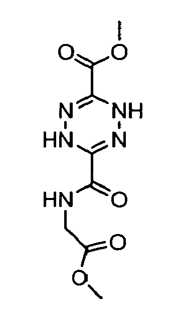

### Beispiel 5

### Reaktionen von Dienen und Dienophilen zur DAR mit inversem Elektronenbedarf

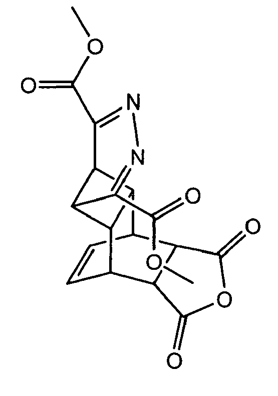

### Umsetzung des tricyclischen Anhydrides mit Tetrazin-3,6-dicarbonsäure-dimethylesters:

Zu einer Suspension von 1 mmol (198 mg) des Tetrazin-3,6-dicarbonsäure-dimethylesters in 5 ml Tetrahydrofuran wurde unter Kühlung die Lösung des tricyclischen Anhydrides in 5 ml des gleichen Lösungsmittels langsam zugetroft. Unter Stickstoffentwicklung bildete sich eine klare rote Lösung, deren Farbe mit den letzten Tropfen des zugefügten Anhydrides nach gelb umschlug. Es wurde eingeengt und der verbliebene Rückstand aus Essigsäure-ethylester umkristallisiert. Ausbeute ist quantitativ, Fp. 165°C. Das Massenspektrum zeigt den Molekülpeak des Anhydrides bei m/e372.

### Nachweis der Diels Alder Reaktion mit inversem Elektronenbedarf an der Festphase:

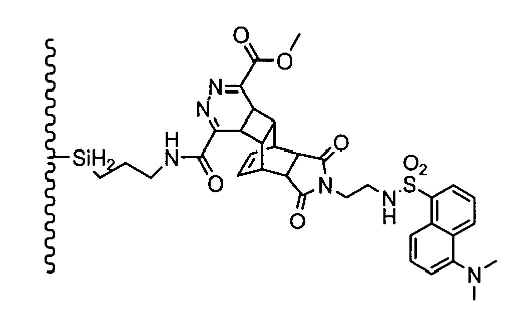

Zunächst wurde 1 gr.Amino-funktionalisiertes Kieselgel (nach Herstellerangaben 1mmol Aminogruppen pro Gramm Kieselgel) in 10 ml Methanol durch Schütteln suspendiert, 2mmol des Dihydrotetrazins-3,6-dicarbonsäureesters zugegeben und bei 60°C für 5 Std. im geschlossenen Gefäß geschüttelt. Dann wurde abgesaugt, mit Methanol und Ether mehrfach gewaschen und getrocknet. Es wurden 900 mg Kieselgel erhalten. Durch Elementaranalyse konnte durch die Berechnung des C/N-Verhältnisses eine etwa 70%ige Belegung nachgewiesen werden. Oxidation mit Isoamylnitrit.

Das erhaltene Kieselgel wurde in Essigester suspendiert und mit einem 5-fachen Überschuss an Isoamylnitrit für 5 Std bei Raumtemperatur geschüttelt. Es wurde filtriert, mit Essigester und Ether mehrfach gewaschen und getrocknet. Das Kieselgel hatte nunmehr eine leicht rosarote Farbe angenommen. Die Elementaranalyse ergab nur geringfügige Änderungen im C/N-Verhältniss. Die Diels Alder Reaktion wurde mit dem bereits mehrfach benutzten tricyclischen Anhydrid durchgeführt. Dazu wurden 100mg des Tetrazin beladenen Kieselgels in Essigester suspendiert und mit 0,3 mmol des Anhydrides für 2 Std. bei Raumtemperatur geschüttelt. Es wurde wieder filtriert, 5-mal mit Essigester gewaschen und getrocknet. Das C/N-Verhältnis der Elementaranalyse bestätigte die ursprüngliche bestimmte Belegung des Kieselgels mit Dihydrotetrazin mit etwa 70%.

Eine weitere Diels Alder Reaktion wurde mit dem Dansyl-Derivat durchgeführt, dessen Synthese unter Dienophilen bereits beschrieben wurde. Dazu wurden 50 mg des Tetrazin beladenen Kieselgels in Essigester suspendiert und für eine Stunde mit 0,05 mmol des Dansyl-Tricyclus umgesetzt. Es wurde abgesaugt , 10-mal gewaschen und getrocknet. Das erhaltene Kieselgel zeigte im UV-Licht eine starke grüne Fluoreszenz. Zur Kontrolle wurde das Dihydrotetrazin-beladene Kieselgel in gleicher Weise umgesetzt, wobei keine Fluoreszenz am Kieslegel zu beobachten war.

Verankerung des Tricyclus als Dienophil an der Festphase:

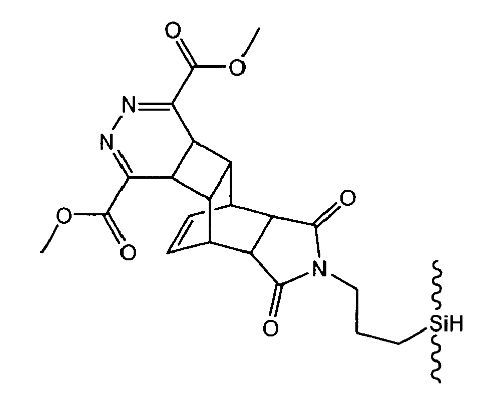

Amino-funktionalisiertes Kieselgel (1gr.) wurde in 10 ml Ethanol suspendiert, 2 mmol (400mg) des tricyclischen Anhydrides zugegeben und 3 Std. bei 80°C geschüttelt. Es wurde über eine Fritte abgesaugt, mehrfach mit Ethanol und abschließend mit Ether gewaschen und getrocknet. Die Bestimmung des C/N-Verhältnisses durch Elementaranalyse ergab einen Belegungsgrad von 70% der vorhandenen Amino-Gruppen.

100mg des funktionalisierten Kieselgels wurde in Tetrahydrofuran suspendiert und mit einer 0,5 molaren Lösung des Tetrazinesters in THF titriert. Die Entfärbung des Tetrazins erfolgt sehr rasch durch die ablaufende Diels Alder Reaktion. Aus dem Verbrauch an Tetrazin konnte wiederum auf eine ursprünglich etwa 70%.ige Belegung geschlossen werden.

In ähnlicher Weise wurden die folgenden Produkte durch Diels Alder Reaktion mit inversem Elektronenbedarf hergestellt, isoliert und charakterisiert:

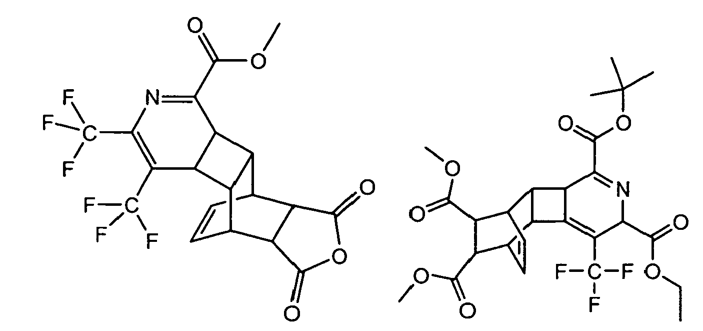

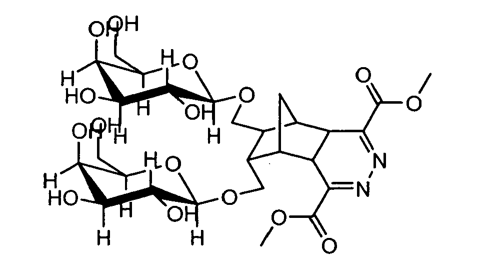

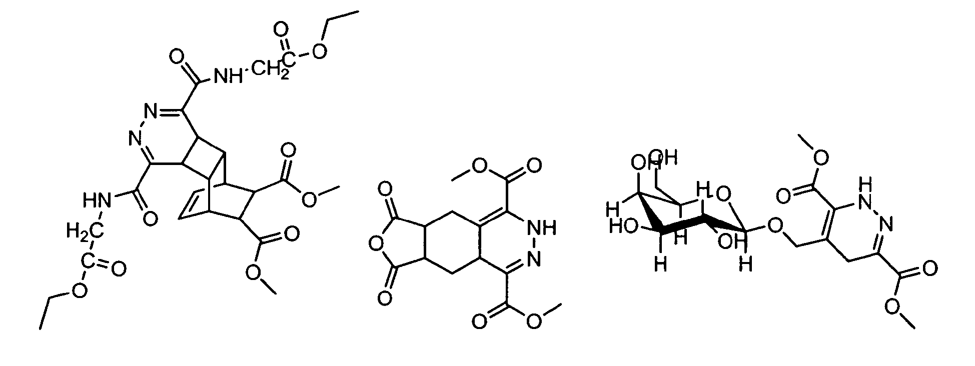

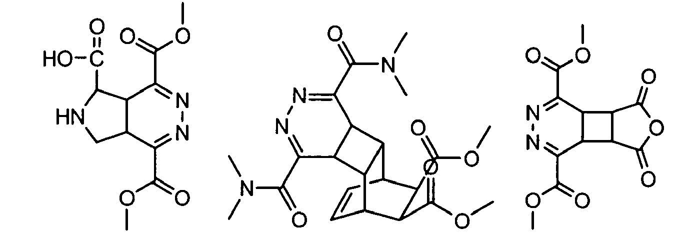

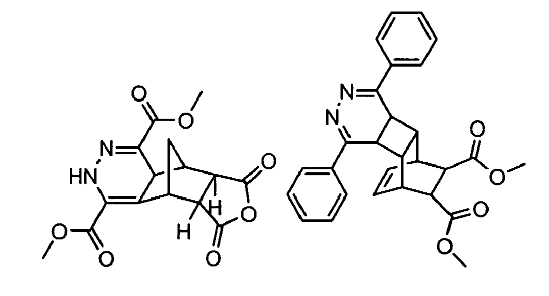

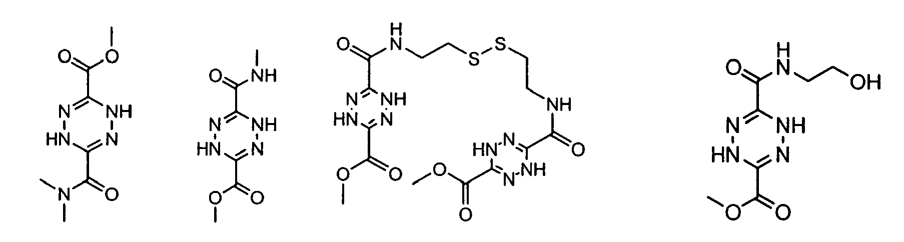

### Beispiel 6

### Funktionalisierung der Triazine

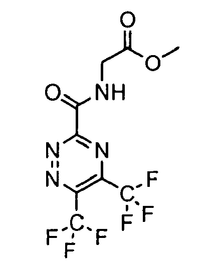

### Umsetzung des 5,6-Bis-trifluormethyl-triazin-3-carbonsäuremethylesters mit Glycinmethylester

Glycinmethylester Hydrochlorid 3 mmol (375 mg) wurden in 10 ml Dioxan suspendiert und dann 3 mmol Triethylamin (0,42 ml) zugegeben. Nach 30 Min. wurde dann 2 mmol (550 mg) des Bis-trifluormethyl-triazin-carbonsäureesters in 10 ml Dioxan zugegeben und für 5 Std. bei 80°C gehalten. Es wurde eingeengt und der Rückstand an Kieselgel mit Hexan/Essigester chromatographiert. Es wurden 400 mg des Produktes erhalten. Die Masse bestätigt die Struktur, wobei die Verbindung offenbar als Mono-Hydrat vorliegt.

Die folgenden Verbindungen wurden in analoger Weise hergestellt und charakterisiert:

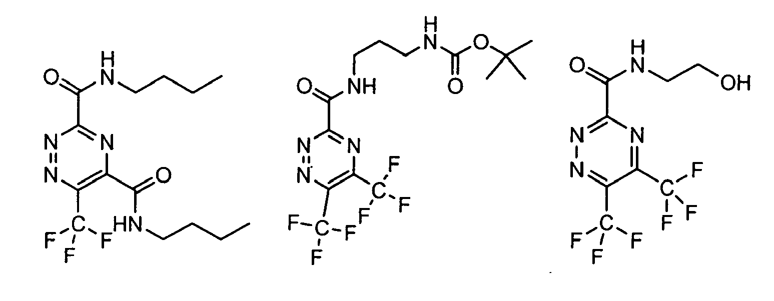

Diels Alder Reaktion der Triazine

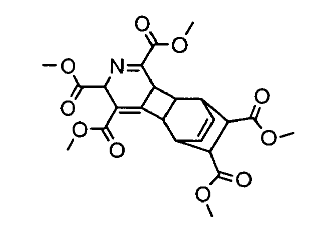

Der Triazin-tri-carbonsäuremethylester 1 mmol (255 mg) wurde in 2 ml THF gelöst und tropfenweise mit einer Lösung von 1 mmol (248 mg) des tricyclischen Dicarbonsäure-dimethylesters in 1 ml THF versetzt. Es wird Stickstoff-Entwicklung beobachtet und eine Farbaufhellung. Nach 2 Std. bei Raumtemperatur wird eingeengt und der Rückstand an Kieselgel mit Hexan/Essigester 1:1 chromatographiert. Es werden 250 mg des Adduktes isoliert entspr 50% Ausbeute. Das Massenspektrum bestätigt die Struktur, Molekülpeak bei m/e 475.

## Patentansprüche

1. Verfahren zur Verknüpfung zweier Moleküle mittels Diels-Alder-Reaktion mit inversem Elektronenbedarf (DARinv), das die folgenden Schritte aufweist:
Umsetzung eines
(a) Diazins, Triazins oder Tetrazins mit einem oder mehreren elektronenziehenden Substituenten am Ring als Dienkomponente, wobei die elektronenziehenden Substutuenten ausgewählt sind aus:
- COOR
- C(O)NR₂
- CX₃ (X = Halogen)
- Halogen
- CN
- SO₂-R oder SO₃-R
- PR₂
mit R = H, Alkyl, Aryl-, Heterocyclus, wobei diese wiederum ggf. substituiert sein können mit Alkyl-, OH-, SH-, Halogen-, Aryl-, Heterocyclus, Nitro-, Carboxyamido-, oder Amin-Gruppe mit
(b) einer isolierten Doppelbindung bzw. Dreifachbindung in einem (hetero)carbocyclischen Ring oder einer isolierten olefinischen Doppelbindung bzw. Dreifachbindung in einer linearen oder verzweigten Kohlenwasserstoffkette, die ggf. Heteroatome enthalten kann, als Dienophilkomponente

2. Verfahren nach Anspruch 1, wobei an die Dien- und/oder Dienophil-Komponente eine oder mehrere Aminosäure-, Peptid-, Saccharid-, Lipid- oder Oligonukleotid- bzw. Nukleinsäure-Substituenten, pharmazeutische Wirkstoffe, Markierungen, Farbstoffe, Komplexe, Quantumdots, Chelat-/Kompexbildner, Diagnostika oder Therapeutika gekoppelt sind.

3. Verfahren nach Anspruch 1 oder 2, wobei die Dien und/oder Dienophil-Komponente eine oder mehrere Funktionalitäten unterschiedlicher Reaktivität zur Durchführung der Diels-Alder-Reaktion enthält.

4. Verfahren nach einem der Ansprüche 1 bis 3" wobei das Dien ausgewählt ist aus: Tetrazinmonoamid, Tetrazindiamid, Tetrazin-3-trifluormethyl-6-carbonsäureamid, Triazin-tricarbonsäure-monoamid, Triazin-tricarbonsäurediamid, Triazin-tricarbonsäure-triamid, oder 1,2-Diazin-3,6-dicarbonsäureamid.

5. Verfahren nach Anspruch 4, wobei das Dien 3-Carboxymethyl-5,6-bis-trifluormethyl-triazin-1,2,4. oder 1,2,4,5-Tetrazin-dicarbonsäure-dimethylester ist.

6. Verfahren nach Anspruch 1, 2 oder 3, wobei das Dienophil ausgewählt ist aus: exo- bzw. endo-Norbornendicarbonsäureanhydrid, Cyclobutendicarbonsäureanhydrid, Cyclohexendicarbonsäureanhydrid" Dehydroprolin, Allyl-Prolin, Allylmalonester, Allylgalactose, Allyl-Silsesquioxan, oder Dicyclopentadienon.

7. Verfahren nach Anspruch 4, wobei die Tetrazinverbindungen aus den entsprechenden Dihydroverbindungen durch nachfolgende Oxidation gewonnen werden.

8. Verfahren nach einem der Ansprüche 1-7, wobei die Dien- und Dienophil-Komponente über einem Linker miteinander verbunden sind.

9. Verfahren nach einem der Ansprüche 1-8, wobei die Diels-Alder-Reaktion mit inversem Elektronenbedarf in wässriger oder alkoholischer Lösung bei 20-100°C durchgeführt wird.
